# FASCICULE DE BREVET EUROPEEN

(11) **EP 4 010 381 B1**
(45) Date de publication et mention de la délivrance du brevet: **16.04.2025**
(21) Numéro de dépôt: 20760896.9
(22) Date de dépôt: 06.08.2020
(51) Int. Cl.: C08C 19/22, C07D 233/32, C08F 236/10, C08F 136/08, C08L 9/00, C08L 9/06, C08L 15/00

(54) **POLYMERE PORTANT DES GROUPES PENDANTS FONCTIONNELS PARTICULIERS IMIDAZOLIDINONE N-SUBSTITUÉS**
POLYMER MIT SPEZIFISCHEN N-SUBSTITUIERTEN IMIDAZOLIDINONANHÄNGENDEN FUNKTIONELLEN GRUPPEN
POLYMER HAVING SPECIFIC N-SUBSTITUTED IMIDAZOLIDINONE PENDANT FUNCTIONAL GROUPS

(30) Priorité: 07.08.2019 FR 1909029
(43) Date de publication de la demande: 15.06.2022
(73) Titulaire: COMPAGNIE GENERALE DES ETABLISSEMENTS MICHELIN, 63000 Clermont-Ferrand (FR)
(72) Inventeur: JEAN-BAPTISTE-DIT-DOMINIQUE, François, 63040 CLERMONT-FERRAND Cedex 9 (FR); FLEURY, Etienne, 63040 CLERMONT-FERRAND CEDEX 9 (FR); GANDER, Sophie, 63040 CLERMONT-FERRAND CEDEX 9 (FR)
(74) Mandataire: M.F.P. Michelin
(86) Numéro de dépôt international: PCT/FR2020/051439
(87) Numéro de publication internationale: WO 2021/023948

(56) Documents cités:
- WO-A1-2019/007883
- WO-A1-2019/007884

## Description

La présente invention concerne des nouveaux polymères, notamment des élastomères diéniques, modifiés par un groupement azoté non-associatif. Ces nouveaux polymères modifiés sont notamment utilisables dans des compositions de caoutchouc, en particulier destinées à la fabrication de pneumatiques.

Dans le domaine industriel des objets fabriqués à partir de compositions de caoutchouc, notamment des pneumatiques, des mélanges de polymères avec des charges renforçantes sont souvent utilisés. Pour que de telles compositions présentent de bonnes propriétés, on recherche en permanence des moyens pour améliorer la dispersion des charges renforçantes au sein de ces polymères. En effet, une bonne dispersion de ces charges au sein de ces polymères est souvent synonyme d'une hystérèse faible et donc d'une résistance au roulement améliorée lorsque ces compositions de caoutchouc sont utilisées dans des pneumatiques.

Or, l'amélioration de la résistance au roulement est un challenge permanent pour les manufacturiers de pneumatiques depuis que les économies de carburant et la nécessité de protéger l'environnement sont devenues une priorité.

De nombreuses solutions ont été proposées pour atteindre l'objectif de baisse de l'hystérèse.

En particulier, on peut citer la modification de la structure des polymères, notamment des élastomères diéniques, en fin de polymérisation au moyen d'agents de modification, le but étant d'atteindre une bonne interaction entre le polymère ainsi modifié et la charge renforçante, qu'il s'agisse de noir de carbone ou d'une charge inorganique renforçante telle que de la silice. On peut citer par exemple le document EP0778311B1 qui décrit l'utilisation de polymères diéniques fonctionnalisés par un groupement silanol en extrémité de chaîne. Plus récemment, la demande de brevet WO2009/077837A1 décrit des élastomères fonctionnalisés par un groupement silanol à une extrémité de chaîne et par un groupement aminé à l'autre extrémité de chaîne. Ces polymères modifiés sont efficaces pour réduire l'hystérèse.

Cependant, les manufacturiers de pneumatiques cherchent toujours à réduire l'hystérèse d'une composition de caoutchouc.

C'est la raison pour laquelle, des recherches ont été menées sur d'autres réactions de modification de polymères, notamment d'élastomères diéniques, en vue de l'obtention de compositions de caoutchouc possédant une diminution de l'hystérèse. A ce titre, on peut mentionner l'utilisation de composés portant des groupements associatifs azotés comprenant au moins un motif les rendant susceptibles de s'associer entre eux par des liaisons hydrogène pour modifier les polymères utilisés dans les compositions de caoutchouc. Ces polymères modifiés porteurs de fonctions imidazolidinone non-substituées sont décrits dans le document WO2019/007884A1.

Cependant, il existe chez les manufacturiers de pneumatiques un besoin permanent de toujours continuer à diminuer encore plus l'hystérèse de compositions de caoutchouc et c'est l'objectif de la présente invention.

Ce but est atteint en ce que la demanderesse a découvert, de manière surprenante, au cours de ses recherches qu'un élastomère diénique modifié par un agent de modification comprenant un groupement azoté non-associatif de type imidazolidinone N-substitué confère aux compositions le contenant une diminution inattendue de l'hystérèse.

L'invention a pour objet un polymère modifié obtenu par greffage d'au moins un composé de formule (I) sur au moins une insaturation de la chaîne d'un polymère initial
dans laquelle : 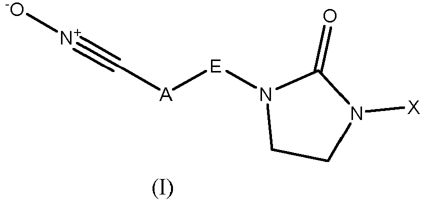
- A représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ;
- E représente un groupe divalent hydrocarboné comprenant éventuellement un ou plusieurs hétéroatomes ; et
- X désigne un atome d'halogène, de préférence un atome de chlore.

Le polymère modifié de l'invention comprend au moins un groupement imidazolidinone N-substitué, c'est-à-dire un groupement azoté non-associatif, pendant le long de sa chaîne. Le polymère modifié de l'invention présente l'avantage de conférer aux compositions le contenant, une diminution significative de l'hystérèse, c'est-à-dire une amélioration de la performance résistance au roulement. Un autre objet de la présente invention concerne une composition comprenant le polymère modifié tel que décrit ci-dessus et au moins un additif.

Un autre objet de l'invention concerne un polymère modifié tel que défini ci-dessus, caractérisé en ce qu'il est en outre obtenu par greffage d'au moins un composé de formule (V) sur au moins une autre insaturation de la chaîne dudit polymère initial
dans laquelle : 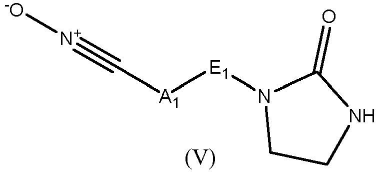
- A1 représente un cycle arènediyle, éventuellement substitué par une ou plusieurs chaînes hydrocarbonées, identiques ou différentes, aliphatiques, de préférence saturée, linéaires ou ramifiées, éventuellement substituées ou interrompues par un ou plusieurs hétéroatomes ; et
- E1 représente un groupe divalent hydrocarboné comprenant éventuellement un ou plusieurs hétéroatomes.

Ce polymère modifié de l'invention comprend au moins un groupement imidazolidinone N-substitué, c'est-à-dire un groupement azoté non-associatif, pendant le long de sa chaîne et au moins un groupement imidazolidinone non-substitué, c'est-à-dire un groupement azoté associatif, pendant le long de sa chaîne. Le polymère modifié de l'invention présente l'avantage de conférer aux compositions le contenant, de manière surprenante, une amélioration du compromis rigidité à cru/ hystérèse.

Un autre objet de la présente invention concerne une composition comprenant au moins un polymère modifié tel que défini ci-dessus et au moins un additif.

Un premier objet de l'invention concerne un polymère modifié obtenu par greffage d'au moins un composé de formule (I) sur au moins une insaturation de la chaîne d'un polymère initial 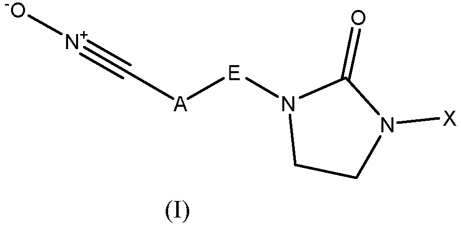 dans laquelle :
- A représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ;
- E représente un groupe divalent hydrocarboné comprenant éventuellement un ou plusieurs hétéroatomes ; et
- X désigne un atome d'halogène, de préférence un atome de chlore.

Le polymère modifié de l'invention comprend au moins un groupement imidazolidinone N-substitué, c'est-à-dire un groupement azoté non-associatif, pendant le long de sa chaîne. Ce polymère modifié présente l'avantage de conférer aux compositions le comprenant une diminution de l'hystérèse, c'est-à-dire une amélioration de la performance résistance au roulement.

Dans la présente, sauf indication expresse différente, tous les pourcentages (%) indiqués sont des pourcentages (%) en masse.

D'autre part, tout intervalle de valeurs désigné par l'expression « entre a et b » représente le domaine de valeurs allant de plus de a à moins de b (c'est-à-dire bornes a et b exclues) tandis que tout intervalle de valeurs désigné par l'expression « de a à b » signifie le domaine de valeurs allant de a jusqu'à b (c'est-à-dire incluant les bornes strictes a et b).

Les composés mentionnés dans la description peuvent être d'origine fossile ou biosourcés. Dans ce dernier cas, ils peuvent être, partiellement ou totalement, issus de la biomasse ou obtenus à partir de matières premières renouvelables issues de la biomasse.

Par « groupement associatif », on entend des groupes susceptibles de s'associer notamment les uns aux autres par des liaisons hydrogène, ioniques et/ou hydrophobes. Il s'agit, selon un mode préféré de l'invention, de groupes susceptibles de s'associer par des liaisons hydrogène. Lorsque les groupes associatifs sont susceptibles de s'associer par des liaisons hydrogène, chaque groupe associatif comporte de préférence au moins un site donneur et un site accepteur vis-à-vis de la liaison hydrogène de sorte que deux groupes associatifs identiques sont auto-complémentaires et peuvent s'associer entre eux en formant au moins deux liaisons hydrogène. Les groupes associatifs selon l'invention sont également susceptibles de s'associer par des liaisons hydrogène, ioniques et/ou hydrophobes à des fonctions présentes sur des charges, notamment sur les charges renforçantes et en particulier sur les charges renforçantes inorganiques, telles que la silice.

Par « groupement non associatif », on entend des groupes qui ne sont pas aptes à s'associer notamment les uns aux autres par des liaisons hydrogène, ioniques et/ou hydrophobes. Préférentiellement, il s'agit de groupes qui ne sont pas aptes à s'associer par des liaisons hydrogène. Ces groupes ne comportent pas de site donneur et de site accepteur vis-à-vis de la liaison hydrogène de sorte qu'aucun de ces groupes identiques ne sont auto-complémentaires. Ils ne peuvent pas s'associer entre eux en formant au moins deux liaisons hydrogène.

Par « hétéroatome », sauf mention contraire, on entend un atome choisi dans le groupe constitué par un atome de soufre, un atome d'oxygène et un atome d'azote.

Par « polymère modifié obtenu par greffage » ou « polymère modifié par greffage », on entend un polymère comportant des fonctions, notamment des fonctions imidazolidinone N-substituées, qui ont été introduites dans la chaîne du polymère. En pratique, le polymère modifié est obtenu par réaction de greffage d'au moins un composé portant des fonctions N-imidazolidinone substituées et portant une fonction apte à former une liaison covalente avec une insaturation de la chaîne du polymère, cette fonction étant un oxyde de nitrile. Lors de la réaction de greffage, la ou les fonctions oxyde de nitrile du composé à greffer forment des liaisons covalentes avec la ou les insaturations de la chaîne du polymère.

De manière connue, un polymère comprend généralement au moins une chaîne polymère principale. Cette chaîne polymère peut être qualifié de principale à partir du moment où toutes les autres chaînes du polymère sont considérées comme des chaînes pendantes comme le mentionne le document « Glossary of basic terms in polymer science » (IUPAC recommandations 1996), PAC, 1996, 68, 2287, p2294.

Par « insaturation » on entend une liaison covalente multiple entre deux atomes de carbone ; cette liaison covalente multiple pouvant être une double liaison carbone-carbone ou une triple liaison carbone-carbone, de préférence une double liaison carbone-carbone.

Par « chaîne de polymère initial », on entend au sens de la présente invention la chaîne du polymère avant la réaction de greffage ; cette chaîne comprenant au moins une insaturation susceptible, plus préférentiellement au moins deux insaturations susceptibles, de réagir avec les composés ayant une fonction oxyde de nitrile, tels que décrits ci-après. Le polymère initial est donc le polymère servant de réactif de départ lors de la réaction de greffage. La réaction de greffage permet à partir d'un polymère initial d'obtenir un polymère modifié.

Le polymère initial est un élastomère diénique.

Par élastomère (ou indistinctement caoutchouc) « diénique », qu'il soit naturel ou synthétique, doit être compris de manière connue un élastomère constitué au moins en partie (i.e., un homopolymère ou un copolymère) d'unités monomères diènes (monomères porteurs de deux doubles liaisons carbone-carbone, conjuguées ou non).

Ces élastomères diéniques peuvent être classés dans deux catégories : « essentiellement insaturés » ou « essentiellement saturés ». On entend en général par « essentiellement insaturé », un élastomère diénique issu au moins en partie de monomères diènes conjugués, ayant un taux de motifs ou unités d'origine diénique (diènes conjugués) qui est supérieur à 15 % (% en moles); c'est ainsi que des élastomères diéniques tels que les caoutchoucs butyle ou les copolymères de diènes et d'alpha-oléfines type EPDM n'entrent pas dans la définition précédente et peuvent être notamment qualifiés d'élastomères diéniques « essentiellement saturés » (taux de motifs d'origine diénique faible ou très faible, toujours inférieur à 15 %).

On entend particulièrement par élastomère diénique susceptible d'être utilisé dans le cadre de la présente invention :
- tout homopolymère d'un monomère diène, conjugué ou non, ayant de 4 à 18 atomes de carbone ;
- tout copolymère d'un diène, conjugué ou non, ayant de 4 à 18 atomes de carbone et d'au moins un autre monomère.

L'autre monomère peut être l'éthylène, une oléfine ou un diène, conjugué ou non.

À titre de diènes conjugués conviennent les diènes conjugués ayant de 4 à 12 atomes de carbone, en particulier les 1,3-diènes, tels que notamment le 1,3-butadiène et l'isoprène.

À titre de diènes non conjugués conviennent les diènes non conjugués ayant de 6 à 12 atomes de carbone, tels que le 1,4-hexadiène, l'éthylidène norbornène, le dicyclopentadiène.

À titre d'oléfines conviennent les composés vinylaromatiques ayant de 8 à 20 atomes de carbone et les α-monooléfines aliphatiques ayant de 3 à 12 atomes de carbone.

À titre de composés vinylaromatiques conviennent par exemple le styrène, l'ortho-, méta-, para-méthylstyrène, le mélange commercial « vinyle-toluène », le para-tertiobutylstyrène.

À titre d'α-monooléfines aliphatiques conviennent notamment les α-monooléfines aliphatiques acycliques ayant de 3 à 18 atomes de carbone.

Plus particulièrement, l'élastomère diénique peut être :
- tout homopolymère d'un monomère diène conjugué, notamment tout homopolymère obtenu par polymérisation d'un monomère diène conjugué ayant de 4 à 12 atomes de carbone ;
- tout copolymère obtenu par copolymérisation d'un ou plusieurs diènes conjugués entre eux ou avec un ou plusieurs composés vinylaromatiques ayant de 8 à 20 atomes de carbone ;
- un copolymère d'isobutène et d'isoprène (caoutchouc butyle), ainsi que les versions halogénées, en particulier chlorées ou bromées, de ce type de copolymère ;
- tout copolymère obtenu par copolymérisation d'un ou plusieurs diènes, conjugués ou non, avec l'éthylène, une α-monooléfine ou leur mélange comme par exemple les élastomères obtenus à partir d'éthylène, de propylène avec un monomère diène non conjugué du type précité.

Préférentiellement, le polymère initial est, de préférence un élastomère diénique, choisi dans le groupe constitué par les copolymères d'éthylène-propylène-monomère diène (EPDM), le caoutchouc butyle (IRR), le caoutchouc naturel (NR), les polyisoprènes de synthèse (IR), les polybutadiènes (BR), les copolymères de butadiène, les copolymères d'isoprène et les mélanges de ces élastomères.

Préférentiellement, le polymère initial est, de préférence un élastomère diénique, choisi dans le groupe constitué par les copolymères d'éthylène-propylène-monomère diène (EPDM), le caoutchouc butyle (IRR), le caoutchouc naturel (NR), les polyisoprènes de synthèse (IR), les polybutadiènes (BR), les copolymères de butadiène-styrène (SBR), les copolymères d'éthylène-butadiène (EBR), les copolymères d'isoprène-butadiène (BIR) ou les copolymères d'isoprène-butadiène-styrène (SBIR), les copolymères d'isobutène-isoprène (caoutchouc butyle - IIR), les copolymères d'isoprène-styrène (SIR) et les mélanges de ces élastomères.

Préférentiellement, le polymère initial est, de préférence un élastomère diénique, choisi dans le groupe constitué par les copolymères d'éthylène-propylène-monomère diène, le caoutchouc butyle et le mélange de ces caoutchoucs.

Préférentiellement, le polymère initial est, de préférence un élastomère diénique, choisi dans le groupe constitué par le caoutchouc naturel, les polyisoprènes de synthèse, les polybutadiènes, les copolymères de butadiène, les copolymères d'isoprène et les mélanges de ces élastomères.

Plus préférentiellement, le polymère initial est, de préférence un élastomère diénique, choisi dans le groupe constitué par le caoutchouc naturel, les polyisoprènes de synthèse, les polybutadiènes, les copolymères de butadiène-styrène, les copolymères d'éthylène-butadiène, les copolymères d'isoprène-butadiène, les copolymères d'isoprène-butadiène-styrène, les copolymères d'isobutène-isoprène, les copolymères d'isoprène-styrène et les mélanges de ces élastomères.

Préférentiellement, le polymère initial est, de préférence un élastomère diénique, est choisi dans le groupe constitué par les polybutadiènes, les copolymères de butadiène, les copolymères d'isoprène et les mélanges de ces élastomères.

Plus préférentiellement, le polymère initial est un élastomère diénique choisi dans le groupe constitué par les polybutadiènes, les copolymères de butadiène-styrène, les copolymères d'éthylène-butadiène, les copolymères d'isoprène-butadiène, les copolymères d'isoprène-butadiène-styrène, les copolymères d'isobutène-isoprène, les copolymères d'isoprène-styrène et les mélanges de ces élastomères.

Conviennent les polybutadiènes et en particulier ceux ayant une teneur (% molaire) en unités -1,2 comprise entre 4% et 80% ou ceux ayant une teneur (% molaire) en cis-1,4 supérieure à 80%, les polyisoprènes, les copolymères de butadiène-styrène et en particulier ceux ayant une Tg (température de transition vitreuse (Tg, mesurée selon ASTM D3418-08) comprise entre 0°C et - 90°C et plus particulièrement entre - 10°C et - 70°C, une teneur en styrène comprise entre 1% et 60% en poids et plus particulièrement entre 20% et 50%, une teneur (% molaire) en liaisons -1,2 de la partie butadiènique comprise entre 4% et 75%, une teneur (% molaire) en liaisons trans-1,4 comprise entre 10% et 80%, les copolymères de butadiène-isoprène et notamment ceux ayant une teneur en isoprène comprise entre 5% et 90% en poids et une Tg de - 40°C à - 80°C, les copolymères isoprène-styrène et notamment ceux ayant une teneur en styrène comprise entre 5 % et 50 % en poids et une Tg comprise entre - 5 C et - 50°C. Dans le cas des copolymères de butadiène-styrène-isoprène conviennent notamment ceux ayant une teneur en styrène comprise entre 5 % et 50 % en poids et plus particulièrement comprise entre 10 % et 40 %, une teneur en isoprène comprise entre 15 % et 60 % en poids et plus particulièrement entre 20 % et 50 %, une teneur en butadiène comprise entre 5 % et 50 % en poids et plus particulièrement comprise entre 20 % et 40 %, une teneur (% molaire) en unités -1,2 de la partie butadiènique comprise entre 4 % et 85 %, une teneur (% molaire) en unités trans -1,4 de la partie butadiènique comprise entre 6 % et 80 %, une teneur (% molaire) en unités -1,2 plus -3,4 de la partie isoprènique comprise entre 5 % et 70 % et une teneur (% molaire) en unités trans -1,4 de la partie isoprènique comprise entre 10 % et 50 %, et plus généralement tout copolymère butadiène-styrène-isoprène ayant une Tg comprise entre - 5°C et - 70°C.

Les polymères initiaux utilisables dans le cadre de l'invention, de préférence les élastomères, plus préférentiellement les élastomères diéniques, peuvent avoir toute microstructure qui est fonction des conditions de polymérisation utilisées. Ces polymères, peuvent être par exemple à blocs, statistiques, séquencés, microséquencés, et être préparer en dispersion en émulsion ou en solution. Ils peuvent être couplés et/ou étoilés, par exemple au moyen d'un atome silicium ou d'étain qui lie entre elles les chaînes polymères.

Comme vu précédemment, l'élastomère diénique initial est modifié par greffage d'un composé de formule (I) aussi appelé agent de modification : 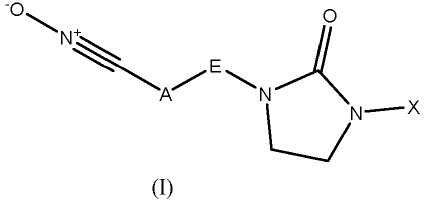 dans laquelle :
- A représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ;
- E représente un groupe divalent hydrocarboné comprenant éventuellement un ou plusieurs hétéroatomes ; et
- X désigne un atome d'halogène, de préférence un atome de chlore.

A représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes.

On entend au sens de la présente invention par « cycle arènediyle », un groupe hydrocarboné aromatique monocyclique ou polycyclique, dérivé d'un arène dans lequel deux atomes d'hydrogène ont été supprimés. Un cycle arènediyle est donc un groupe divalent.

Par « groupe hydrocarboné aromatique monocyclique ou polycyclique », on entend au sens de la présente invention un ou des cycles aromatiques dont le squelette est constitué d'atomes de carbone. Autrement dit, il n'y a pas d'hétéroatomes dans le squelette du cycle. Le cycle arènediyle peut être monocyclique, c'est-à-dire constitué d'un seul cycle, ou polycyclique, c'est-à-dire constitué de plusieurs cycles hydrocarbures aromatiques condensés ; de tels cycles condensés ont alors en commun au moins deux atomes de carbone successifs. Ces cycles peuvent être ortho-condensés ou ortho- et péri-condensés.

De préférence, le cycle arènediyle comprend de 6 à 14 atomes de carbone.

Le cycle arènediyle peut être non substitué, partiellement substitué ou totalement substitué. Un cycle arènediyle est partiellement substitué lorsqu'un ou deux ou plusieurs atomes d'hydrogène (mais pas tous les atomes) sont remplacés par une ou deux ou plusieurs chaînes hydrocarbonées, aliphatiques, de préférence saturées, linéaires ou ramifiées, éventuellement substituées par un ou plusieurs hétéroatomes. Lesdites chaînes sont aussi appelées substituants. Si tous les atomes d'hydrogène sont remplacés par lesdites chaînes, alors le cycle arènediyle est totalement substitué. Les substituants du cycle arènediyle peuvent être identiques ou différents les uns par rapport aux autres.

De préférence, lorsque le cycle arènediyle, est substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes, cette ou ces chaînes peuvent être inertes vis-à-vis de la fonction imidazolidinone N-substituée et de l'oxyde de nitrile.

On entend, au sens de la présente invention, par « chaîne hydrocarbonée inerte vis-à-vis de la fonction imidazolidinone N-substituée et de l'oxyde de nitrile » une chaîne hydrocarbonée qui ne réagit ni avec ladite fonction imidazolidinone N-substituée ni avec ledit oxyde de nitrile. Ainsi, ladite chaîne hydrocarbonée inerte par rapport à ladite fonction imidazolidinone N-substituée et audit oxyde de nitrile est, de préférence, une chaîne hydrocarbonée aliphatique qui ne présente pas de fonctions alcényle ou alcynyle, susceptibles de réagir avec ladite fonction ou ledit groupement, c'est-à-dire est une chaîne hydrocarbonée aliphatique saturée, linéaire ou ramifiée, éventuellement substituée ou interrompue par un ou plusieurs hétéroatomes, et comprenant préférentiellement de 1 à 24 atomes de carbone.

De préférence, le groupement A est un cycle arènediyle en C6-C14 éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes. Plus préférentiellement, le groupement A est un cycle arènediyle en C6-C14, éventuellement substitué par une ou plusieurs chaînes hydrocarbonées, identiques ou différentes, saturées, en C1-C24, linéaires ou ramifiées, éventuellement substituées ou interrompues par un ou plusieurs hétéroatomes d'azote, de soufre ou d'oxygène. Plus préférentiellement encore, le groupement A est un cycle arènediyle en C6-C14, éventuellement substitué par un ou plusieurs substituants, identiques ou différents, choisis dans le groupe constitué par un alkyle en C1-C12, (plus préférentiellement en C1-C6, plus préférentiellement encore en C1-C4), un groupement OR', un groupement -NHR', un groupement -SR' où R' est un groupe alkyle en C1-C12, plus préférentiellement en C1-C6, plus préférentiellement encore en C1-C4.

De préférence, le composé de formule (I) est choisi parmi les composés de formule (Ia) et (Ib) 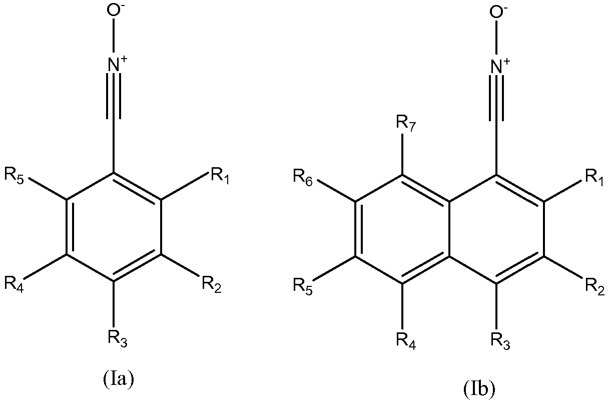 dans lesquelles :
- un groupement choisi parmi R1 à R5 de la formule (Ia) et un groupement choisi parmi R1 à R7 de la formule (Ib) désignent le groupe de formule (II) suivante : 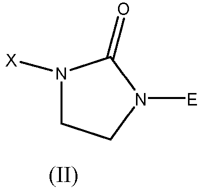 dans laquelle E et X sont tels que définis précédemment,
- les quatre groupements de la formule (Ia) choisis parmi R1 à R5 autres que celui désignant le groupe de formule (II) et les six groupements de la formule (Ib) choisis parmi R1 à R7 autres que celui désignant le groupe de formule (II), identiques ou différents, représentent indépendamment les uns des autres, un atome d'hydrogène ou une chaîne hydrocarbonée aliphatique, de préférence saturée, linéaire ou ramifiée, éventuellement substituée ou interrompue par un ou plusieurs hétéroatomes.

Préférentiellement, dans les composés de formule (Ia) et (Ib), les quatre groupements de la formule (Ia) choisis parmi R1 à R5 autres que celui désignant le groupe de formule (II) et les six groupements de la formule (Ib) choisis parmi R1 à R7 autres que celui désignant le groupe de formule (II), identiques ou différents, représentent indépendamment les uns des autres, un atome d'hydrogène ou une chaîne hydrocarbonée aliphatique, saturée, linéaire ou ramifiée, en C1-C24, éventuellement substituée ou interrompue par un ou plusieurs hétéroatomes.

Plus préférentiellement encore, dans les composés de formule (Ia) et (Ib), les quatre groupements de la formule (Ia) choisis parmi R1 à R5 autres que celui désignant le groupe de formule (II) et les six groupements de la formule (Ib) choisis parmi R1 à R7 autres que celui désignant le groupe de formule (II), identiques ou différents, sont choisis dans le groupe formé par un atome d'hydrogène, un alkyle en C1-C12 (plus préférentiellement en C1-C6, plus préférentiellement encore en C1-C4), un groupement
-OR', un groupement -NHR' et un groupement -SR' où R' est un alkyle en C1-C12, plus préférentiellement en C1-C6, plus préférentiellement encore en C1-C4.

Selon un mode de réalisation préféré de l'invention, dans la formule (Ia), R2 représente un groupe de formule (II) tel que défini ci-dessus et R1, R3, R4 et R5, identiques ou différents, représentent un atome d'hydrogène ou une chaîne hydrocarbonée aliphatique, de préférence saturée, linéaire ou ramifiée, en C1-C24, éventuellement substituée ou interrompue par un ou plusieurs hétéroatomes. Plus préférentiellement, R2 représente un groupe de formule (II) tel que défini ci-dessus et R1, R3, R4 et R5, identiques ou différents, sont choisis dans le groupe constitué par un atome d'hydrogène, un alkyle en C1-C12 (plus préférentiellement en C1-C6, plus préférentiellement encore en C1-C4), un groupement
-OR', un groupement -NHR' et un groupement -SR' où R' est un alkyle en C1-C12, plus préférentiellement en C1-C6, plus préférentiellement encore en C1-C4.

Plus préférentiellement encore dans ce mode de réalisation, R2 représente un groupe de formule (II) tel que défini ci-dessus, R4 représente un atome d'hydrogène et R1, R3 et R5 représentent une chaîne hydrocarbonée aliphatique, de préférence saturée, linéaire ou ramifiée, en C1-C24, éventuellement substituée ou interrompue par un ou plusieurs hétéroatomes. Plus préférentiellement encore, R2 représente un groupe de formule (II) tel que défini ci-dessus, R4 représente un atome d'hydrogène et R1, R3 et R5, identiques ou différents, sont choisis dans le groupe constitué par un alkyle en C1-C12 (plus préférentiellement en C1-C6, plus préférentiellement encore en C1-C4), un groupement-OR', un groupement -NHR' et un groupement -SR' où R' est un alkyle en C1-C12, plus préférentiellement en C1-C6, plus préférentiellement encore en C1-C4.

Selon un autre mode de réalisation préférée de l'invention, dans la formule (Ib), R1 représente un groupe de formule (II) tel que défini ci-dessus et R2 à R7 identiques ou différents, représentent un atome d'hydrogène ou une chaîne hydrocarbonée aliphatique, de préférence saturée, linéaire ou ramifiée, en C1-C24, éventuellement substituée ou interrompue par un ou plusieurs hétéroatomes. Plus préférentiellement, R1 représente un groupe de formule (II) tel que défini ci-dessus et R2 à R7, identiques ou différents, sont choisis dans le groupe constitué par un atome d'hydrogène, un alkyle en C1-C12 (plus préférentiellement en C1-C6, plus préférentiellement encore en C1-C4), un groupement -OR', un groupement -NHR' et un groupement -SR' où R' est un alkyle en C1-C12, plus préférentiellement en C1-C6, plus préférentiellement encore en C1-C4. Plus préférentiellement encore dans ce mode de réalisation, R1 représente un groupe de formule (II) tel que défini ci-dessus et R2 à R7, identiques, représentent un atome d'hydrogène.

Dans les composés de formule (I), (Ia) et (Ib), le groupement E est un groupe divalent hydrocarboné pouvant éventuellement contenir un ou plusieurs hétéroatome(s). Par « groupe divalent hydrocarboné » on entend au sens de la présente invention, un groupe espaceur (ou un groupe de liaison) formant un pont entre le groupement A et le groupe imidazolidinone N-substitué, ce groupe espaceur étant une chaîne hydrocarbonée, saturée ou insaturée, de préférence saturée, en C1-C24, linéaire ou ramifiée, pouvant éventuellement contenir un ou plusieurs hétéroatome(s) tel(s) que par exemple N, O et S. Ladite chaîne hydrocarbonée peut éventuellement être substituée, pour autant que les substituants ne réagissent pas avec l'oxyde de nitrile et le groupe imidazolidinone N-substitué tel que défini ci-dessus.

Préférentiellement, dans les composés de formule (I), (Ia) et (Ib), le groupement E est une chaîne hydrocarbonée, linéaire ou ramifiée, de préférence saturée, en C1-C24, plus préférentiellement en C1-C10, encore plus préférentiellement en C1-C6, éventuellement interrompue par un ou plusieurs atomes d'azote, de soufre ou d'oxygène.

De préférence, dans les composés de formule (I), (Ia) et (Ib), le groupement E est choisi dans le groupe constitué par -R-, -NH-R-, -O-R- et -S-R- où R est un alkylène, linéaire ou ramifié, en C1-C24, de préférence en C1-C10, plus préférentiellement en C1-C6.

Plus préférentiellement encore, dans les composés de formule (I), (Ia) et (Ib), le groupement E est choisi dans le groupe constitué par -R- et -OR- où R est un alkylène, linéaire ou ramifié, en C1-C24, de préférence en C1-C10, plus préférentiellement en C1-C6.

Plus préférentiellement encore, dans les composés de formule (I), (Ia) et (Ib), le groupement E est choisi parmi -CH2-, -CH2-CH2-, -CH2-CH2-CH2-, -CH2-CH2-CH2-CH2-, -O-CH2-, -O-CH2-CH2-, -O-CH2-CH2-CH2- et -O-CH2-CH2-CH2-CH2-.

Selon un mode de réalisation préférée, le composé de formule (I) est le composé de formule (Ib).

Selon ce mode de réalisation préféré, on préfère particulièrement le composé de formule (Ib) tel que défini ci-dessus avec le groupement E choisi dans le groupe constitué par -R- et -OR- où R est un alkylène, linéaire ou ramifié, en C1-C24, de préférence en C1-C10, plus préférentiellement en C1-C6 et avec le groupement X qui est un atome de chlore.

Selon ce mode de réalisation, plus préférentiellement encore, le composé de formule (I) est le composé de formule (Ib) avec :
- R1 représente un groupe de formule (II) dans lequel le groupement E est choisi dans le groupe constitué par -R- et -OR- où R est un alkylène, linéaire ou ramifié, en C1-C24, de préférence en C1-C10, plus préférentiellement en C1-C6 et le groupement X désigne un atome de chlore ; et
- R2 à R7 identiques ou différents, représentent un atome d'hydrogène ou une chaîne hydrocarbonée aliphatique, de préférence saturée, linéaire ou ramifiée, en C1-C24, éventuellement substituée ou interrompue par un ou plusieurs hétéroatomes.

Selon ce mode de réalisation, plus préférentiellement encore, le composé de formule (I) est le composé de formule (Ib) avec :
- R1 représente un groupe de formule (II) dans lequel le groupement E est choisi dans le groupe constitué par -R- et -OR- où R est un alkylène, linéaire ou ramifié, en C1-C24, de préférence en C1-C10, plus préférentiellement en C1-C6 et le groupement X désigne un atome de chlore ; et
- R2 à R7, identiques ou différents, sont choisis dans le groupe constitué par un atome d'hydrogène, un alkyle en C1-C12 (plus préférentiellement en C1-C6, plus préférentiellement encore en C1-C4), un groupement -OR', un groupement -NHR' et un groupement -SR' où R' est un alkyle en C1-C12, plus préférentiellement en C1-C6, plus préférentiellement encore en C1-C4.

Selon ce mode de réalisation, plus préférentiellement encore, le composé de formule (I) est le composé de formule (Ib) avec :
- R1 représente un groupe de formule (II) dans lequel le groupement E est choisi dans le groupe constitué par -R- et -OR- où R est un alkylène, linéaire ou ramifié, en C1-C24, de préférence en C1-C10, plus préférentiellement en C1-C6 et le groupement X désigne un atome de chlore ; et
- R2 à R7, identiques, représentent un atome d'hydrogène.

Selon un mode de réalisation particulier, le composé de formule (I) est choisi dans le groupe constitué par le composé de formule (III) et de formule (IV), plus préférentiellement est le composé de formule (III)

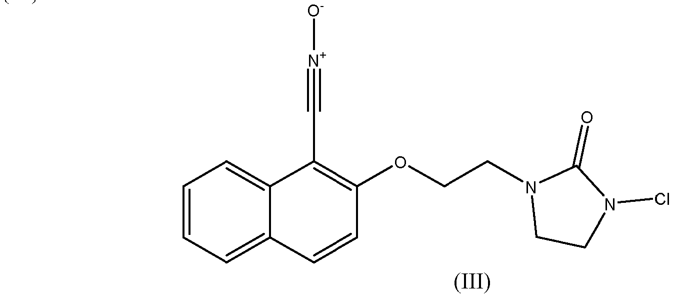

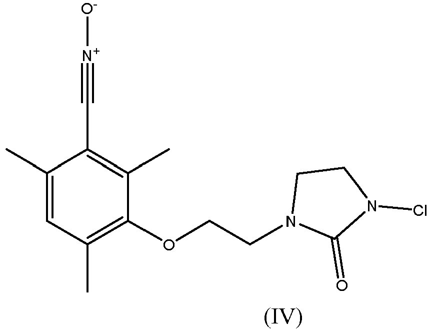

Les composés de formule (I), en particulier ceux de formule (Ia), (Ib), (III) et (IV) peuvent être obtenu à partir d'un procédé de synthèse comprenant les étapes successives suivantes :
(b1) la réaction d'un composé de formule (IX) suivante : 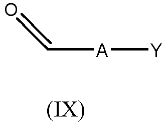
   dans laquelle A est tel que défini précédemment et Y représente un groupe nucléophile, avec un composé de formule (X) suivante : 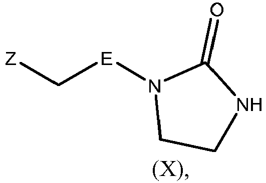
   dans laquelle E est tel que défini précédemment, et Z représente un groupe nucléofuge ;
   en présence d'au moins un solvant polaire S1, d'au moins une base, à une température T1 allant de 70 à 150°C, pour former un composé de formule (XI) suivante : 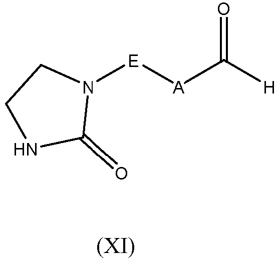
(b2) la réaction dudit composé de formule (XI) avec une solution aqueuse d'hydroxylamine à une température T2 allant de 30 à 70°C, pour obtenir un composé oxime de formule (XII) suivante : 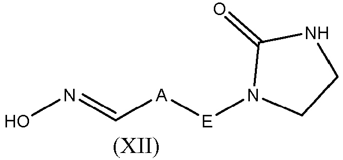
(c) une étape de récupération dudit composé oxime de formule (XII) ;
(d) une étape d'oxydation du composé oxime de formule (XII) avec un agent oxydant, en présence d'au moins un solvant organique S2, le taux de l'agent oxydant étant d'au moins 6 équivalents molaire par rapport à la quantité molaire de composé oxime de formule (XII).

On entend au sens de la présente demande par « solvant polaire » un solvant présentant une constante diélectrique supérieure à 2,2.

On entend au sens de la présente demande « par groupe nucléofuge » un groupe partant qui emporte son doublet de liaison.

On entend au sens de la présente demande « par groupe nucléophile » un composé comprenant au moins un atome porteur d'un doublet libre ou un atome chargé négativement.

Comme expliqué précédemment, le procédé de synthèse du composé de formule (I) comprend notamment les étapes successives (b1) et (b2).

Selon un mode de réalisation particulier, les deux étapes (b1) et (b2) sont séparées par une étape d'isolation et de purification du composé de formule (XI).

Selon un autre mode de réalisation, les deux étapes (b1) et (b2) sont réalisées selon une synthèse monotope, c'est-à-dire que les étapes (b1) et (b2) sont « one pot » (procédé de synthèse monotope en deux étapes), soit sans isolation du composé de formule (XI) intermédiaire.

Le procédé comprend une étape (b1) de réaction d'un composé de formule (IX), telle que mentionnée ci-dessus, porteur d'un groupe Y, avec un composé de formule (X), telle que mentionnée ci-dessus, porteur d'un groupe Z.

De manière préférée, le groupe Y est choisi parmi les fonctions hydroxyle, thiol et amine primaire ou secondaire.

Le groupe Z peut être choisi parmi le chlore, le brome, l'iode, le groupe mésylate, le groupe tosylate, le groupe acétate et le groupe trifluorométhylsulfonate.

Ladite étape (b1) du procédé est réalisée en présence d'au moins un solvant polaire S1, et d'au moins une base, à une température T1 allant de 70 à 150°C.

Selon un mode de réalisation particulier du procédé, le solvant polaire S1 est un solvant polaire miscible dans l'eau, préférentiellement un solvant protique.

Le diméthylformamide (DMF), le diméthylsulfoxyde (DMSO), la 1,3-diméthyl-2-imidazolidinone (DMI), la 1,3-diméthyl-3,4,5,6-tetrahydro-2(1H)-pyrimidinone (DMPU), l'isopropanol, l'acétonitrile, l'éthanol, le n-butanol et le n-propanol sont des exemples de solvants S1 utilisables dans le procédé.

De préférence, le solvant protique est alcoolique.

Avantageusement, le composé de formule (IX) représente de 5 à 40% en poids, de préférence de 10 à 30% en poids, par rapport au poids du solvant.

La base peut être choisie parmi les alcoolates alcalins, les carbonates alcalins, les carbonates alcalino-terreux, les hydroxydes alcalins, les hydroxydes alcalino-terreux et leurs mélanges.

Avantageusement, il est possible d'ajouter :
- un ou plusieurs catalyseurs choisis parmi un catalyseur de type sel d'argent (I), un catalyseur de transfert de phase de type ammonium quaternaire, et leurs mélanges ;
- un ou plusieurs liquides ioniques.

Préférentiellement, la base est choisie parmi le méthanolate de sodium, le carbonate de potassium et la soude, plus préférentiellement le carbonate de potassium.

Selon un mode de réalisation particulier du procédé, la quantité molaire de base est de 1,5 à 8 équivalents molaire, de préférence de 2 à 6 équivalents molaire, par rapport à la quantité molaire de composé de formule (IX).

Comme expliqué précédemment, l'étape (b1) du procédé est réalisée à une température T1 allant de 70 à 150°C.

De préférence, la température T1 est une température allant de 70 à 120°C, plus préférentiellement de 80 à 110°C.

Comme expliqué précédemment, l'étape (b1) du procédé est suivie de l'étape (b2) de l'ajout dans le milieu réactionnel contenant le composé de formule (XI) d'une solution aqueuse d'hydroxylamine à une température T2 allant de 30 à 70°C.

Préférentiellement, l'ajout de la solution aqueuse d'hydroxylamine est réalisé lorsque la conversion du composé de formule (IX) est d'au moins 70% en poids.

Avantageusement, la température T2 varie de 40 à 60°C.

Le procédé comprend également une étape (c) de récupération, telle que mentionnée ci-avant, du composé oxime de formule (XII).

De manière préférée, le composé oxime de formule (XII) est récupéré par précipitation à l'eau, suivie éventuellement d'un lavage à l'eau.

Le procédé comprend également une étape (d) d'oxydation du composé oxime de formule (XII) avec un agent oxydant pour donner le composé de formule (I), en particulier les composés préférés, en présence d'au moins un solvant organique S2 ; la quantité d'agent oxydant est d'au moins 6 équivalents molaire, préférentiellement de 6,5 à 15 équivalents molaire, par rapport à la quantité molaire de composé oxime de formule (XII).

Cette quantité d'agent oxydant peut être ajoutée en une fois ou en plusieurs fois au cours de l'étape (d), de préférence ajoutée en deux fois au cours de l'étape (d).

De manière préférée, ledit agent oxydant est choisi parmi l'hypochlorite de sodium, le N-bromosuccinimide en présence d'une base et le N-chlorosuccinimide en présence d'une base, préférentiellement ledit agent oxydant est l'hypochlorite de sodium.

Préférentiellement, le solvant organique S2 est un solvant organique choisi parmi les solvants chlorés et de type ester, éther et alcool, plus préférentiellement choisi parmi le dichlorométhane, l'acétate d'éthyle, l'acétate de butyle, l'éther diéthylique, l'isopropanol et l'éthanol, encore plus préférentiellement choisi parmi l'acétate d'éthyle et l'acétate de butyle.

De préférence, le composé oxime de formule (XII) représente de 1 à 30% en poids, de préférence de 1 à 20% en poids, par rapport au poids total de l'ensemble comprenant ledit composé oxime de formule (XII), ledit solvant organique S2 et ledit agent oxydant. Préférentiellement, ce procédé comprend, après l'étape (d), une étape (e) de récupération du composé de formule (I).

Selon un mode de réalisation particulier, le procédé peut comprendre une étape (a2) de fabrication du composé de formule (X), préalable à l'étape (b1), par mise en réaction d'un composé de formule (XIII) suivante : 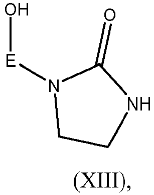 dans laquelle :
- E est tel que défini précédemment,
- avec un agent permettant la formation du groupe nucléofuge Z.

De manière préférée, ledit agent est le chlorure de thionyle.

De préférence, l'étape (a2) est réalisée en l'absence ou en présence d'au moins un solvant S4, préférentiellement un solvant chloré, plus préférentiellement le dichlorométhane.

Avantageusement, l'étape (a2) est immédiatement suivie d'une étape (a3) de récupération du composé de formule (X), préférentiellement par purification au toluène, plus préférentiellement par cristallisation du composé de formule (X) dans le toluène.

L élastomère diénique modifié est obtenu par greffage d'au moins un composé de formule (I), de préférence d'au moins un composé de formule (Ib), plus préférentiellement d'au moins un composé de formule (III), sur au moins une insaturation de la chaîne dudit élastomère diénique initial.

Le greffage du polymère se fait par réaction dudit polymère initial avec l'oxyde de nitrile du composé de formule (I), de préférence du composé de formule (Ib), plus préférentiellement du composé de formule (III). Lors de cette réaction, ce groupement forme une liaison covalente avec la chaîne du polymère. Le greffage du composé de formule (I), de préférence du composé de formule (Ib), plus préférentiellement du composé de formule (III), est effectué par cycloaddition [3+2] de l'oxyde de nitrile et une insaturation de la chaîne du polymère initial. Un mécanisme de la cycloaddition [3+2] est illustré dans le document WO2012/007441.

Selon l'invention, le polymère porte le long de la chaîne polymère principale un ou plusieurs groupes pendants issus de la réaction de greffage du composé de formule (I), de préférence du composé de formule (Ib), plus préférentiellement du composé de formule (III), tels que définis ci-dessus. Avantageusement, ces groupes pendants sont répartis le long de la chaîne polymère principale de façon aléatoire.

Selon un mode de réalisation préféré, le taux molaire de greffage du composé de formule (I), de préférence du composé de formule (Ib), plus préférentiellement du composé de formule (III), est compris dans un domaine allant de 0,01 % à 5 %, de préférence de 0,01 % à 1 %, plus préférentiellement de 0,1 à 1 %.

Par « taux molaire de greffage » on entend le nombre de mol de composé de formule (I), de préférence du composé de formule (Ib), plus préférentiellement du composé de formule (III), greffé sur le polymère pour 100 moles d'unité monomère constituant le polymère. Le taux molaire de greffage peut être déterminé par les méthodes conventionnelles d'analyses des polymères, telles que par exemple l'analyse RMN ¹H.

Le greffage du composé de formule (I), de préférence du composé de formule (Ib), plus préférentiellement du composé de formule (III), sur le polymère initial, peut être réalisé en masse, par exemple dans une extrudeuse, dans un mélangeur interne ou dans un mélangeur externe tel qu'un mélangeur à cylindres. Il peut aussi être réalisé en solution, en continu ou en discontinu. Le polymère ainsi modifié peut être séparé de sa solution par tout type de moyen connu par l'homme du métier et en particulier par une opération de stripping à la vapeur d'eau.

Dans le procédé décrit ci-dessus, le polymère initial est un élastomère diénique, tel que décrit ci-dessus.

Dans la suite de la description, par concision, l'élastomère diénique, modifié par greffage avec le composé de formule (I), de préférence avec le composé de formule (Ib), plus préférentiellement avec le composé de formule (III), est appelé polymère P1.

L'invention a également pour objet une composition comprenant au moins un polymère modifié (polymère P1) tel que décrit ci-dessus et au moins un additif.

Les additifs utilisables dans la composition selon l'invention peuvent être des plastifiants (tels que des huiles plastifiantes et/ou des résines plastifiantes), des charges (renforçantes ou non renforçantes) des pigments, des agents de protection tels que cires anti-ozone, anti-ozonants chimiques, anti-oxydants, des agents anti-fatigue, des résines renforçantes (telles que décrites par exemple dans la demande WO 02/10269), un système de réticulation, par exemple à base de soufre et autres agents de vulcanisation, et/ou de peroxyde et/ou de bismaléimide. De préférence, cet additif est une charge renforçante, plus préférentiellement cet additif est une charge renforçante inorganique, plus préférentiellement encore cet additif est une silice.

Un autre objet de la présente invention est un polymère modifié (dans la suite de la description pour plus de concision ce polymère est appelé polymère P2) obtenu par greffage d'au moins un composé de formule (I) tel que décrit précédemment, de préférence d'au moins un composé de formule (Ib), plus préférentiellement, d'au moins un composé de formule (III) sur au moins une insaturation de la chaîne du polymère initial et d'au moins un composé de formule (V) sur une autre insaturation de la chaîne du polymère initial 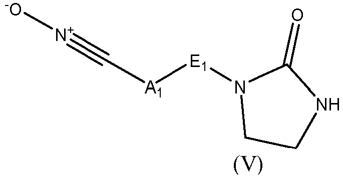 dans laquelle :
- A1 représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ; et
- E1 représente un groupe divalent hydrocarboné comprenant éventuellement un ou plusieurs hétéroatomes.

Le polymère P2 est un élastomère diénique. Le polymère modifié P2 de l'invention comprend au moins un groupement imidazolidinone N-substitué, c'est-à-dire un groupement azoté non-associatif, pendant le long de sa chaîne et au moins un groupement imidazolidinone non-substitué, c'est-à-dire un groupement azoté associatif, pendant le long de sa chaîne. Ce polymère modifié P2 présente l'avantage de conférer aux compositions le contenant, de manière surprenante, une amélioration du compromis rigidité à cru/ hystérése.

De préférence, le groupement A1 du composé de formule (V) est un cycle arènediyle en C6-C14 éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes. Plus préférentiellement, ce groupement A1 est un cycle arènediyle en C6-C14, éventuellement substitué par une ou plusieurs chaînes hydrocarbonées, identiques ou différentes, saturées en C1-C24, linéaires ou ramifiées, éventuellement substituées ou interrompues par un ou plusieurs hétéroatomes d'azote, de soufre ou d'oxygène Plus préférentiellement encore, ce groupement A1 est un cycle arènediyle en C6-C14, éventuellement substitué par un ou plusieurs substituants, identiques ou différents, choisis dans le groupe constitué par un alkyle en C1-C12, (plus préférentiellement en C1-C6, plus préférentiellement encore en C1-C4), un groupement-OR', un groupement -NHR' et un groupement -SR' où R' est un groupe alkyle en C1-C12, plus préférentiellement en C1-C6, plus préférentiellement encore en C1-C4.

De préférence, le composé de formule (V) est choisi parmi les composés de formule (Va) et (Vb) 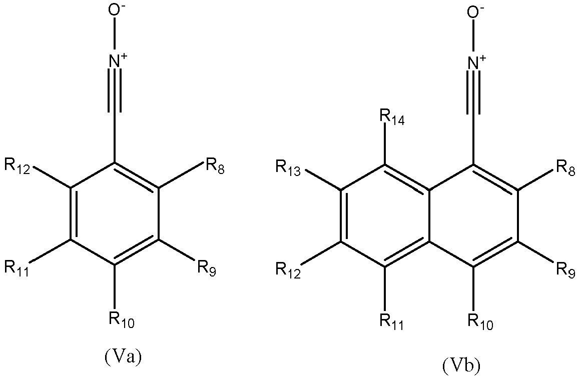 dans lesquelles :
- un groupement choisi parmi R8 à R12 de la formule (Va) et un groupement choisi parmi R8 à R14 de la formule (Vb) désignent le groupe de formule (VI) suivante 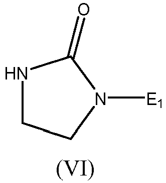 dans laquelle E1 est tel que défini ci-dessus ; et
- les quatre groupements de la formule (Va) choisis parmi R8 à R12 autres que celui désignant le groupe de formule (VI) et les six groupements de la formule (Vb) choisis parmi R8 à R14 autres que celui désignant le groupe de formule (VI), identiques ou différents, représentent indépendamment les uns des autres, un atome d'hydrogène ou une chaîne hydrocarbonée aliphatique, de préférence saturée, linéaire ou ramifiée éventuellement substituée ou interrompue par un ou plusieurs hétéroatomes.

Préférentiellement, dans les composés de formule (Va) et (Vb), les quatre groupements de la formule (Va) choisis parmi R1 à R5 autres que celui désignant le groupe de formule (VI) et les six groupements de la formule (Vb) choisis parmi R1 à R7 autres que celui désignant le groupe de formule (VI), identiques ou différents, représentent indépendamment les uns des autres, un atome d'hydrogène ou une chaîne hydrocarbonée aliphatique, saturée, linéaire ou ramifiée, en C1-C24, éventuellement substituée ou interrompue par un ou plusieurs hétéroatomes.

Plus préférentiellement encore, dans les composés de formule (Va) et (Vb), les quatre groupements de la formule (Va) choisis parmi R1 à R5 autres que celui désignant le groupe de formule (VI) et les six groupements de la formule (Vb) choisis parmi R1 à R7 autres que celui désignant le groupe de formule (VI), identiques ou différents, sont choisis dans le groupe formé par un atome d'hydrogène, un alkyle en C1-C12 (plus préférentiellement en C1-C6, plus préférentiellement encore en C1-C4), un groupement-OR', un groupement -NHR' et un groupement -SR' où R' est un alkyle en C1-C12, plus préférentiellement en C1-C6, plus préférentiellement encore en C1-C4.

Selon un mode de réalisation préférée de l'invention, dans la formule (Va), R9 représente un groupe de formule (VI) tel que défini ci-dessus et R8, R10, R11 et R12, identiques ou différents, représentent un atome d'hydrogène ou une chaîne hydrocarbonée aliphatique, de préférence saturée, linéaire ou ramifiée, en C1-C24, éventuellement substituée ou interrompue par un ou plusieurs hétéroatomes. Plus préférentiellement, R9 représente un groupe de formule (VI) tel que défini ci-dessus et R8, R10, R11 et R12, identiques ou différents sont choisis dans le groupe constitué par un atome d'hydrogène, un alkyle en C1-C12 (plus préférentiellement en C1-C6, plus préférentiellement encore en C1-C4),un groupement -OR', un groupement -NHR' et un groupement -SR' où R' est un alkyle en C1-C12, plus préférentiellement en C1-C6, plus préférentiellement encore en C1-C4.

Plus préférentiellement encore dans ce mode de réalisation, dans la formule (Va), R9 représente un groupe de formule (VI) tel que défini ci-dessus, R11 représente un atome d'hydrogène et R8, R10 et R12 représentent une chaîne hydrocarbonée aliphatique, de préférence saturée, linéaire ou ramifiée en C1-C24, éventuellement substituée ou interrompue par un ou plusieurs hétéroatomes. Plus préférentiellement encore, R9 représente un groupe de formule (VI) tel que défini ci-dessus, R11 représente un atome d'hydrogène et R8, R10 et R12, identiques ou différents, sont choisis dans le groupe constitué par un alkyle en C1-C12 (plus préférentiellement en C1-C6, plus préférentiellement encore en C1-C4), un groupement -OR', un groupement-NHR' et un groupement -SR' où R' est un alkyle en C1-C12, plus préférentiellement en C1-C6, plus préférentiellement encore en C1-C4.

Selon un autre mode de réalisation préférée de l'invention, dans la formule (Vb), R8 représente un groupe de formule (VI) tel que défini ci-dessus et R9 à R14 identiques ou différents, représentent un atome d'hydrogène ou une chaîne hydrocarbonée aliphatique, de préférence saturée, linéaire ou ramifiée, en C1-C24, éventuellement substituée ou interrompue par un ou plusieurs hétéroatomes. Plus préférentiellement, R8 représente un groupe de formule (VI) tel que défini ci-dessus et R9 à R14, identiques ou différents, sont choisis dans le groupe constitué par un atome d'hydrogène, un alkyle en C1-C12 (plus préférentiellement en C1-C6, plus préférentiellement encore en C1-C4), un groupement -OR', un groupement -NHR', un groupement -SR' où R' est un alkyle en C1-C12, plus préférentiellement en C1-C6, plus préférentiellement encore en C1-C4. Plus préférentiellement encore dans ce mode de réalisation, R8 représente un groupe de formule (VI) tel que défini ci-dessus et R9 à R14, identiques, représentent un atome d'hydrogène.

Dans les composés de formule (V), (Va) et (Vb), le groupement E1 est un groupe divalent hydrocarboné pouvant éventuellement contenir un ou plusieurs hétéroatome(s). Par « groupe divalent hydrocarboné » on entend au sens de la présente invention, un groupe espaceur (ou un groupe de liaison) formant un pont entre le groupement A1 et le groupe imidazolidinone, ce groupe espaceur étant une chaîne hydrocarbonée, saturée ou insaturée, de préférence saturée, en C1-C24, linéaire ou ramifiée, pouvant éventuellement contenir un ou plusieurs hétéroatome(s) tel(s) que par exemple N, O et S. Ladite chaîne hydrocarbonée peut éventuellement être substituée, pour autant que les substituants ne réagissent pas avec l'oxyde de nitrile et le groupe imidazolidinone tel que défini ci-dessus.

Préférentiellement, dans les composés de formule (V), (Va) et (Vb), le groupement E1 est une chaîne hydrocarbonée, linéaire ou ramifiée, de préférence saturée, en C1-C24, plus préférentiellement en C1-C10, encore plus préférentiellement en C1-C6, éventuellement interrompue par un ou plusieurs atomes d'azote, de soufre ou d'oxygène.

De préférence, dans les composés de formule (V), (Va) et (Vb), le groupement E1 est choisi dans le groupe constitué par -R-, -NH-R-, -O-R- et -S-R- où R est un alkylène, linéaire ou ramifié, en C1-C24, de préférence en C1-C10, plus préférentiellement en C1-C6.

Plus préférentiellement encore, dans les composés de formule (V), (Va) et (Vb), le groupement E1 est choisi dans le groupe constitué par -R- et -OR- où R est un alkylène, linéaire ou ramifié, en C1-C24, de préférence en C1-C10, plus préférentiellement en C1-C6.

Plus préférentiellement encore, dans les composés de formule (V), (Va) et (Vb), le groupement E1 est choisi parmi -CH2-, -CH2-CH2-, -CH2-CH2-CH2-, -CH2-CH2-CH2-CH2-, -O-CH2-, -O-CH2-CH2-, -O-CH2-CH2-CH2- et -O-CH2-CH2-CH2-CH2-.

Selon un mode de réalisation préférée, le composé de formule (V) est le composé de formule (Vb).

Dans ce mode de réalisation préférée, on préfère en particulier le composé de formule (Vb) tel que défini ci-dessus avec le groupement E1 choisi dans le groupe constitué par -R- et -OR- où R est un alkylène, linéaire ou ramifié, en C1-C24, de préférence en C1-C10, plus préférentiellement en C1-C6.

Selon ce mode de réalisation, plus préférentiellement encore, le composé de formule (V) est le composé de formule (Vb) avec :
- R8 représente un groupe de formule (VI) dans lequel le groupement E1 est choisi dans le groupe constitué par -R- et -OR- où R est un alkylène, linéaire ou ramifié, en C1-C24, de préférence en C1-C10, plus préférentiellement en C1-C6; et
- R9 à R14 identiques ou différents, représentent un atome d'hydrogène ou une chaîne hydrocarbonée aliphatique, de préférence saturée, linéaire ou ramifiée, en C1-C24, éventuellement substituée ou interrompue par un ou plusieurs hétéroatomes.

Selon ce mode de réalisation, plus préférentiellement encore, le composé de formule (V) est le composé de formule (Vb) avec :
- R8 représente un groupe de formule (VI) dans lequel le groupement E1 est choisi dans le groupe constitué par -R- et -OR- où R un alkylène, linéaire ou ramifié, en C1-C24, de préférence en C1-C10, plus préférentiellement en C1-C6; et
- R9 à R14, identiques ou différents, sont choisis dans le groupe formé par un atome d'hydrogène, un alkyle en C1-C12 (plus préférentiellement en C1-C6, plus préférentiellement encore en C1-C4), un groupement -OR', un groupement -NHR', un groupement -SR' où R' est un alkyle en C1-C12, plus préférentiellement en C1-C6, plus préférentiellement encore en C1-C4.

Selon ce mode de réalisation, plus préférentiellement encore, le composé de formule (V) est le composé de formule (Vb) avec :
- R8 représente un groupe de formule (VI) dans lequel le groupement E1 choisi dans le groupe constitué par -R- et -OR- où R un alkylène, linéaire ou ramifié, en C1-C24, de préférence en C1-C10, plus préférentiellement en C1-C6; et
- R9 à R14, identiques, représentent un atome d'hydrogène.

Selon un mode de réalisation préféré, le composé de formule (V) est choisi dans le groupe constitué par le composé de formule (VII) et de formule (VIII), plus préférentiellement encore par le composé de formule (VII)

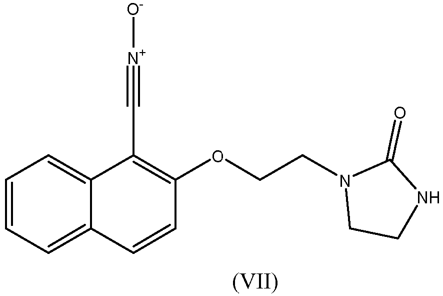

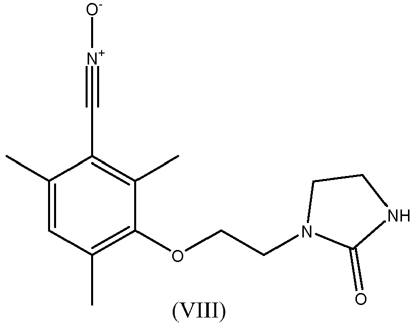

Le composé de formule V, en particulier, les composés de formule (Vb) et (VII), peuvent être, par exemple, synthétisés par le procédé décrit dans le document WO2019007881A1.

L'obtention du polymère P2 s'effectue par greffage du polymère initial avec l'oxyde de nitrile du composé de formule (I) tel que défini ci-dessus et avec l'oxyde de nitrile du composé de formule (V) tel que défini-ci-dessus. Lors de cette réaction, les oxydes de nitrile des composés de formule (I) et de formule (V) forment chacun une liaison covalente avec une insaturation de la chaîne du polymère. Le greffage des composés de formule (I) et (V), est effectué par cycloaddition [3+2] des oxydes de nitrile et des insaturations de la chaîne du polymère initial. Un mécanisme de la cycloaddition [3+2] peut être trouvé dans le document WO2012/007441.

Selon l'invention, le polymère P2 porte le long de la chaîne polymère principale un ou plusieurs groupes pendants issus de la réaction de greffage des composés de formule (I) et (V) tels que définis ci-dessus. Avantageusement, ces groupes pendants sont répartis le long de la chaîne polymère principale de façon aléatoire.

Selon un mode de réalisation préféré, le polymère P2 porte le long de la chaîne polymère principale un ou plusieurs groupes pendants issus de la réaction de greffage des composés de formule (Ib) et (Vb) tels que définis ci-dessus. Avantageusement, ces groupes pendants sont répartis le long de la chaîne polymère principale de façon aléatoire.

Selon un mode de réalisation préféré, le polymère P2 porte le long de la chaîne polymère principale un ou plusieurs groupes pendants issus de la réaction de greffage :
- du composé de formule (Ib) avec R1 représente un groupe de formule (II) dans lequel le groupement E est choisi dans le groupe constitué par -R- et -OR- où R est un alkylène, linéaire ou ramifié, en C1-C24, de préférence en C1-C10, plus préférentiellement en C1-C6, groupement X qui est un atome de chlore ; et R2 à R7, identiques ou différents, sont choisis dans le groupe constitué par un atome d'hydrogène, un alkyle en C1-C12 (plus préférentiellement en C1-C6, plus préférentiellement encore en C1-C4), un groupement -OR', un groupement -NHR' et un groupement -SR' où R' est un alkyle en C1-C12, plus préférentiellement en C1-C6, plus préférentiellement encore en C1-C4 ;et
- du composé de formule (Vb) avec R8 représente un groupe de formule (VI) dans lequel le groupement E1 est choisi dans le groupe constitué par -R- et -OR- où R est un alkylène, linéaire ou ramifié, en C1-C24, de préférence en C1-C10, plus préférentiellement en C1-C6; et R9 à R14, identiques ou différents, sont choisis dans le groupe formé par un atome d'hydrogène, un alkyle en C1-C12 (plus préférentiellement en C1-C6, plus préférentiellement encore en C1-C4), un groupement -OR', un groupement -NHR', un groupement -SR' où R' est un alkyle en C1-C12, plus préférentiellement en C1-C6, plus préférentiellement encore en C1-C4.

Avantageusement, ces groupes pendants sont répartis le long de la chaîne polymère principale de façon aléatoire.

Selon un mode de réalisation encore plus préféré, le polymère P2 porte le long de la chaîne polymère principale un ou plusieurs groupes pendants issus de la réaction de greffage du composé de formule (III) et du composé de formule (VII) tels que définis ci-dessus. Avantageusement, ces groupes pendants sont répartis le long de la chaîne polymère principale de façon aléatoire.

Quel que soit le mode de réalisation, pour le polymère P2, le taux molaire de greffage du composé de formule (I), de préférence du composé de formule (Ib), plus préférentiellement du composé de formule (III), est compris dans un domaine allant de 0,01 % à 5 %, de préférence de 0,01 % à 1 %, plus préférentiellement de 0,1 à 1 % et le taux molaire de greffage du composé de formule (V), de préférence du composé de formule (Vb), plus préférentiellement du composé de formule (VII), est compris dans un domaine allant de 0,01 % à 5 %, de préférence de 0,01 % à 1 %, plus préférentiellement de 0,1 à 1 %.

Le greffage des composés de formule (I) et (V), de préférence des composés de formule (Ib) et (Vb), plus préférentiellement des composés de formule (III) et (VII), sur le polymère initial, peut être réalisé en masse, par exemple dans une extrudeuse, dans un mélangeur interne ou dans un mélangeur externe tel qu'un mélangeur à cylindres. Il peut aussi être réalisé en solution, en continu ou en discontinu. Le polymère ainsi modifié peut être séparé de sa solution par tout type de moyen connu par l'homme du métier et en particulier par une opération de stripping à la vapeur d'eau. Les composés de formule (I) et (V), de préférence des composés de formule (Ib) et (Vb), plus préférentiellement des composés de formule (III) et (VII), peuvent être introduits simultanément dans le mélangeur interne ou externe, ou simultanément dans le réacteur si le greffage s'effectue en solution. Ils peuvent aussi être introduits de manière décalée l'un par rapport à l'autre, l'ordre d'ayant pas d'importance pour le greffage de ces composés sur la chaîne du polymère initial.

L'invention a également pour objet une composition comprenant au moins un polymère modifié, polymère P2, tel que décrit ci-dessus et au moins un additif tel que décrit ci-dessus. De préférence, cet additif est une charge renforçante, plus préférentiellement, cet additif est une charge inorganique renforçante, plus préférentiellement encore cet additif est une silice.

En plus, des objets décrits précédemment, l'invention concerne au moins l'un des objets décrits aux points suivants :
1. Polymère modifié obtenu par greffage d'au moins un composé de formule (I) sur au moins une insaturation de la chaîne d'un polymère initial 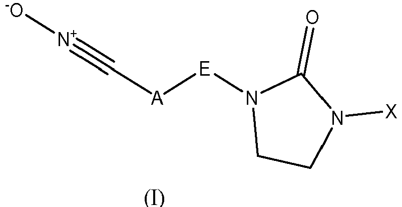 dans laquelle :
   - A représente un cycle arènediyle, éventuellement substitué par une ou plusieurs chaînes hydrocarbonées, identiques ou différentes, aliphatiques, de préférence saturées, linéaires ou ramifiés, éventuellement substituées ou interrompues par un ou plusieurs hétéroatomes ;
   - E représente un groupe divalent hydrocarboné comprenant éventuellement un ou plusieurs hétéroatomes ; et
   - X désigne un atome d'halogène, de préférence un atome de chlore, le polymère initial étant un élastomère diénique.
2. Polymère modifié selon le point 1, dans lequel le polymère initial est choisi dans le groupe constitué par les copolymères d'éthylène-propylène-monomère diène, le caoutchouc butyle, le caoutchouc naturel, les polyisoprènes de synthèse, les polybutadiènes, les copolymères de butadiène, les copolymères d'isoprène et les mélanges de ces élastomères.
3. Polymère modifié selon l'un quelconque des points précédents, dans lequel le taux molaire de greffage du composé de formule (I) est compris dans un domaine allant de 0,01 % à 5 %, de préférence de 0,01 % à 1 %, plus préférentiellement de 0,1 à 1 %.
4. Polymère modifié selon l'un quelconque des points précédents, dans lequel le groupement A est un cycle arènediyle en C6-C14 éventuellement substitué par une ou plusieurs chaînes hydrocarbonées, identiques ou différentes, aliphatiques, de préférences, linéaires ou ramifiées, éventuellement substituées ou interrompues par un ou plusieurs hétéroatomes.
5. Polymère modifié selon l'un quelconque des points précédents, dans lequel le composé de formule (I) est choisi parmi les composés de formule (Ia) et (Ib)
   dans lesquelles : 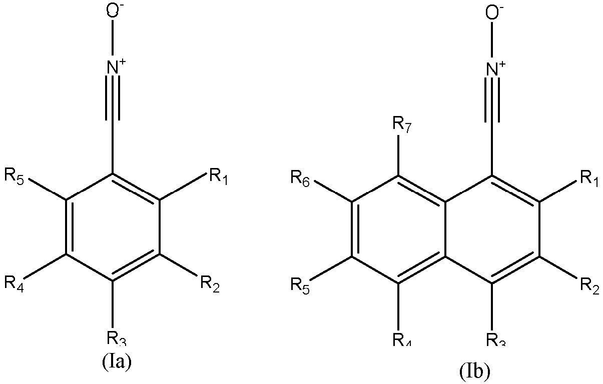
   - un groupement choisi parmi R1 à R5 de la formule (Ia) et un groupement choisi parmi R1 à R7 de la formule (Ib) désignent le groupe de formule (II) suivante : 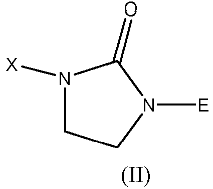 dans laquelle E et X sont tels que défini au point 1,
   - les quatre groupements de la formule (Ia) choisis parmi R1 à R5 autres que celui désignant le groupe de formule (II) et les six groupements de la formule (Ib) choisis parmi R1 à R7 autres que celui désignant le groupe de formule (II), identiques ou différents, représentent indépendamment les uns des autres, un atome d'hydrogène ou une chaîne hydrocarbonée aliphatique, de préférence saturée, linéaire ou ramifiée, éventuellement substituée ou interrompue par un ou plusieurs hétéroatomes.
6. Polymère modifié selon l'un quelconque des points précédents, dans lequel le groupement E est une chaîne hydrocarbonée, linéaire ou ramifiée, de préférence saturée, en C1-C24, de préférence en C1-C10, plus préférentiellement en C1-C6, éventuellement interrompue par un ou plusieurs atomes d'azote, de soufre ou d'oxygène.
7. Polymère modifié selon l'un quelconque des points précédents, dans lequel groupement E est choisi dans le groupe constitué par -R- et -OR- où R est un alkylène, linéaire ou ramifié, en C1-C24, de préférence en C1-C10, plus préférentiellement en C1-C6.
8. Polymère modifié selon l'un quelconque des points 5 à 7, dans lequel le composé de formule (I) est le composé de formule (Ib).
9. Polymère modifié selon l'un quelconque des points précédents, dans lequel le composé de formule (I) est le groupe constitué par le composé de formule (III) 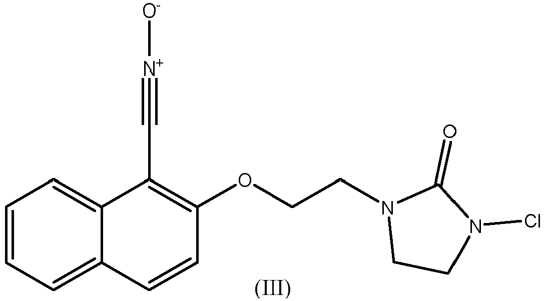
10. Composition comprenant au moins un polymère modifié selon l'un quelconque des points précédents et au moins un additif, de préférence l'additif est une charge, plus préférentiellement encore l'additif est une charge renforçante, encore plus préférentiellement l'additif est une charge renforçante inorganique, plus préférentiellement l'additif est une silice.
11. Bande de roulement comprenant au moins une composition telle que définie au point 10.
12. Pneumatique comprenant au moins une composition telle que définie au point 10.
13. Pneumatique comprenant au moins une bande de roulement telle que définie au point 11.
14. Polymère modifié selon l'un quelconque des points 1 à 9, caractérisé en ce qu'il est en outre obtenu par greffage d'au moins un composé de formule (V) sur au moins une autre insaturation de la chaîne dudit polymère initial 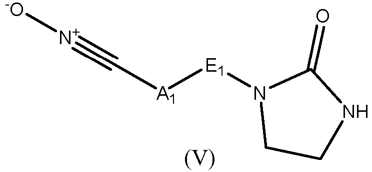 dans laquelle :
   - A1 représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ; et
   - E1 représente un groupe divalent hydrocarboné comprenant éventuellement un ou plusieurs hétéroatomes.
15. Polymère modifié selon le point 14, dans lequel le taux molaire de greffage du composé de formule (V) est compris dans un domaine allant de 0,01 % à 5 %, de préférence de 0,01 % à 1 %, plus préférentiellement de 0,1 à 1 %.
16. Polymère modifié selon l'un quelconque des points 14 à 15, dans lequel le groupement A1 du composé de formule (V) est un cycle arènediyle en C6-C14 éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes.
17. Polymère modifié selon l'un quelconque des points 14 à 16, dans lequel le composé de formule (V) est choisi parmi les composés de formule (Va) et (Vb) 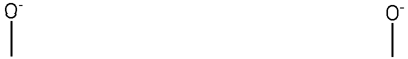 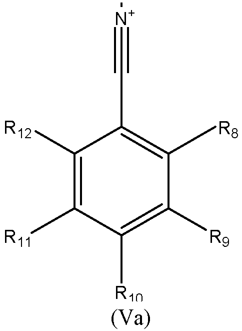 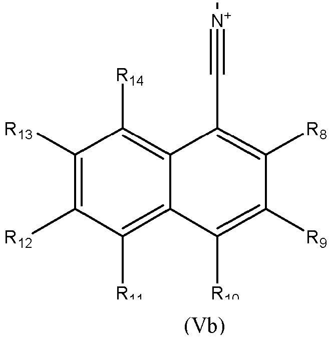 dans lesquelles :
   - un groupement choisi parmi R8 à R12 de la formule (Va) et un groupement choisi parmi R8 à R14 de la formule (Vb) désignent le groupe de formule (II) suivante 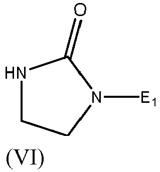 avec E1 tel que défini au point 14 ; et
   - les quatre groupements de la formule (Va) choisis parmi R8 à R12 autres que celui désignant le groupe de formule (VI) et les six groupements de la formule (Vb) choisis parmi R8 à R14 autres que celui désignant le groupe de formule (VI), identiques ou différents, représentent indépendamment les uns des autres, un atome d'hydrogène ou une chaîne hydrocarbonée aliphatique, de préférence saturée, linéaire ou ramifiée, éventuellement substituée ou interrompue par un ou plusieurs hétéroatomes.
18. Polymère modifié selon l'un quelconque des points 14 à 17, dans lequel le groupement E1 du composé de formule (V) ou de formule (VI) est une chaîne hydrocarbonée, linéaire ou ramifiée, de préférence saturée, en C1-C24, de préférence en C1-C10, plus préférentiellement en C1-C6, éventuellement interrompue par un ou plusieurs atomes d'azote, de soufre ou d'oxygène.
19. Polymère modifié selon l'un quelconque des points 14 à 18, dans lequel le groupement E1 du composé de formule (V) ou de formule (VI) est choisi dans le groupe constitué par -R- et -OR- où R est un alkylène, linéaire ou ramifié, en C1-C24, de préférence en C1-C10, plus préférentiellement en C1-C6.
20. Polymère modifié selon l'un quelconque des points 14 à 19, dans lequel le composé de formule (V) est le composé de formule (Vb).
21. Polymère modifié selon l'un quelconque des points 14 à 20, dans lequel le composé de formule (V) est le composé de formule (VII) 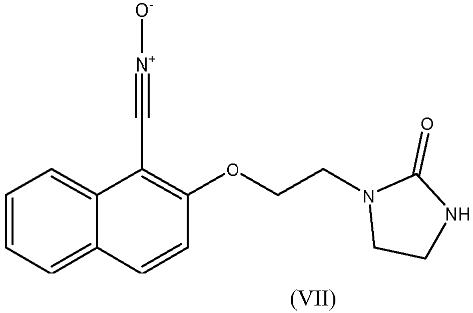
22. Composition comprenant au moins un polymère modifié tel que défini à l'un quelconque des points 14 à 20 et au moins un additif, de préférence l'additif est une charge, plus préférentiellement encore l'additif est une charge renforçante, encore plus préférentiellement l'additif est une charge renforçante inorganique, plus préférentiellement l'additif est une silice.
23. Bande de roulement comprenant au moins une composition telle que définie au point 22.
24. Pneumatique comprenant au moins une composition telle que définie au point 22.
25. Pneumatique comprenant au moins une bande de roulement telle que définie au point 23.
26. Mélange de polymères modifiés, comprenant au moins deux polymères choisis parmi :
   ▪ un polymère P1 défini selon l'un quelconque des points 1 à 9,
   ▪ un polymère P2 défini selon l'un quelconque des points 14 à 21,
   ▪ un polymère P3 obtenu par greffage d'au moins un composé de formule (V) sur au moins une insaturation de la chaîne d'un polymère initial dans laquelle :
      - A1 représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ;
      - E1 représente un groupe divalent hydrocarboné comprenant éventuellement un ou plusieurs hétéroatomes ; et les polymères initiaux des polymères modifiés P1 et P3 pouvant être identiques ou différents ; et à la condition que ledit mélange comprenne au moins ledit polymère P1 ou ledit polymère P2.
27. Mélange de polymères modifiés selon le point 26, dans lequel les polymères initiaux des polymères P1 et P3 sont des élastomères, de préférence des élastomères diéniques, identiques ou différents.
28. Mélange de polymères modifiés selon le point 27, dans lequel les élastomères diéniques sont choisis dans le groupe constitué par les copolymères d'éthylène-propylène-monomère diène, le caoutchouc butyle, le caoutchouc naturel, les polyisoprènes de synthèse, les polybutadiènes, les copolymères de butadiène, notamment les copolymères d'éthylène et de butadiène, les copolymères d'isoprène et les mélanges de ces élastomères. 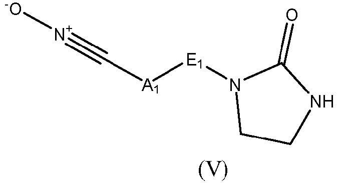
29. Mélange de polymères modifiés selon l'un quelconque des points 26 à 28, dans lequel le taux molaire de greffage du composé de formule (V) sur le polymère P3 est compris dans un domaine allant de 0,01 % à 5 %, de préférence de 0,01 % à 1 %, plus préférentiellement de 0,1 à 1 %.
30. Mélange de polymères modifiés selon l'un quelconque des points 26 à 29, dans lequel le groupement A1 du composé de formule (V) sur le polymère P3 est un cycle arènediyle en C6-C14 éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes.
31. Mélange de polymères modifiés selon l'un quelconque des points 26 à 30, dans lequel le composé de formule (V) sur le polymère P3 est choisi parmi les composés de formule (Va) et (Vb) 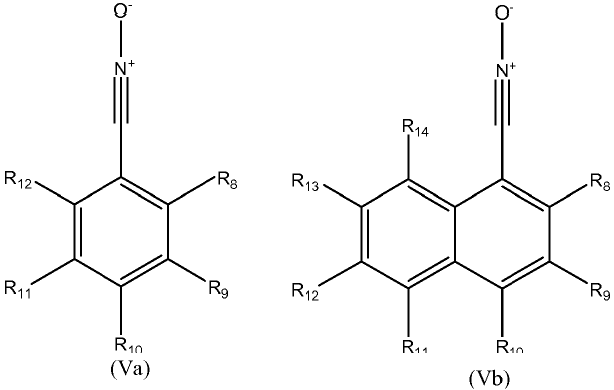 dans lesquelles :
   - un groupement choisi parmi R8 à R12 de la formule (Va) et un groupement choisi parmi R8 à R14 de la formule (Vb) désigne le groupe de formule (VI) suivante 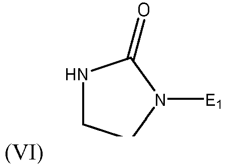 avec E1 tel que défini au point 27, et
   - les autres groupements de la formule (Va) choisis parmi R8 à R12 autres que celui désignant le groupe de formule (VI) et les six groupements de la formule (Vb) choisis parmi R8 à R14 autres que celui désignant le groupe de formule (VI) , identiques ou différents, représentent indépendamment les uns des autres, un atome d'hydrogène ou une chaîne hydrocarbonée aliphatique, de préférence saturée, linéaire ou ramifiée, éventuellement substituée ou interrompue par un ou plusieurs hétéroatomes.
32. Mélange de polymères modifiés selon l'un quelconque des points 26 à 31, dans lequel groupement E1 du composé de formule (V) ou de formule (VI) du polymère P3 est une chaîne hydrocarbonée, linéaire ou ramifiée, de préférence saturée, en C1-C24, de préférence en C1-C10, plus préférentiellement en C1-C6, éventuellement interrompue par un ou plusieurs atomes d'azote, de soufre ou d'oxygène.
33. Mélange de polymères modifiés selon l'un quelconque des points 26 à 32, dans lequel groupement E1 du composé de formule (V) ou de formule (VI) du polymère P3 est choisi dans le groupe constitué par -R- et -OR- où R est un alkylène, linéaire ou ramifié, en C1-C24, de préférence en C1-C10, plus préférentiellement en C1-C6.
34. Mélange de polymères modifiés selon l'un quelconque des points 26 à 33, dans lequel le composé de formule (V) est choisi dans le groupe constitué par le composé de formule (VII) et de formule (VIII), plus préférentiellement le composé de formule (VII) 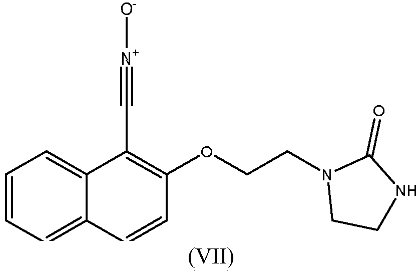 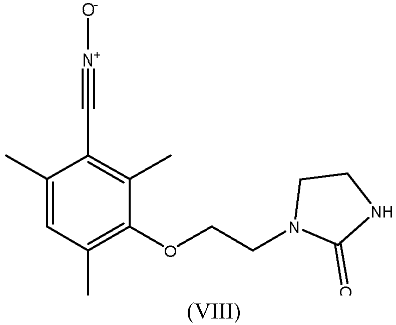
35. Mélange de polymères modifiés selon l'un quelconque des points 26 à 34, dans lequel :
   - le polymère P1 est un polymère modifié, tel que défini ci-dessus, obtenu par greffage d'au moins un composé de formule (Ia) ;
   - le polymère P2, tel que défini ci-dessus, obtenu par greffage d'au moins un composé de formule (Ia) et au moins d'un composé de formule (Va) ; et
   - le polymère P3 est un polymère modifié, tel que défini ci-dessus, obtenu par greffage d'au moins un composé de formule (Va).
36. Mélange de polymères modifiés selon l'un quelconque des points 26 à 34, dans lequel :
   - le polymère P1 est un polymère modifié, tel que défini ci-dessus, obtenu par greffage d'au moins un composé de formule (Ib) ;
   - le polymère P2, tel que défini ci-dessus, obtenu par greffage d'au moins un composé de formule (Ib) et au moins d'un composé de formule (Vb) ; et
   - le polymère P3 est un polymère modifié, tel que défini ci-dessus, obtenu par greffage d'au moins un composé de formule (Vb).
37. Composition comprenant au moins un mélange de polymères modifiés tel que défini à l'un quelconque des points 26 à 36 et au moins un additif, de préférence l'additif est une charge, plus préférentiellement encore l'additif est une charge renforçante, encore plus préférentiellement l'additif est une charge renforçante inorganique, plus préférentiellement l'additif est une silice.
38. Bande de roulement comprenant au moins une composition telle que définie au point 37.
39. Pneumatique comprenant au moins une composition telle que définie au point 37.
40. Pneumatique comprenant au moins une bande de roulement telle que définie au point 38.

### EXEMPLES :

Les exemples qui suivent permettent d'illustrer l'invention, cette dernière ne saurait cependant être limitée à ces seuls exemples.

### 1 Tests et mesures utilisés

### 1.1 Détermination de la température de transition vitreuse

La température de transition vitreuse Tg des polymères sont mesures au moyen d'un calorimètre différentiel (« Differential Scanning Calorimeter ») selon la norme ASTM D3418 (2015).

### 1.2 Mesure des masses molaires moyennes en nombre (Mn), en poids (Mw) et de l'indice de polydispersité pour les élastomères

On utilise la chromatographie d'exclusion stérique ou SEC (Size Exclusion Chromatography). La SEC permet de séparer les macromolécules en solution suivant leur taille à travers des colonnes remplies d'un gel poreux. Les macromolécules sont séparées suivant leur volume hydrodynamique, les plus volumineuses étant éluées en premier.

Sans être une méthode absolue, la SEC permet d'appréhender la distribution des masses molaires d'un élastomère. À partir de produits étalons commerciaux, les différentes masses molaires moyennes en nombre (Mn) et en poids (Mw) peuvent être déterminées et l'indice de polymolécularité (Ip = Mw/Mn) calculé via un étalonnage dit de MOORE.

### Préparation de l'échantillon d'élastomère à tester:

Il n'y a pas de traitement particulier de l'échantillon de l'élastomère avant analyse. Celui-ci est simplement solubilisé à une concentration d'environ 1 g/L, dans du chloroforme ou dans le mélange suivant : tétrahydrofurane + 1 % vol. de diisopropylamine + 1 % vol. de triéthylamine + 1 % vol. d'eau distillée (% vol. = % volume). Puis, la solution est filtrée sur filtre de porosité 0,45 µm avant injection.

### Analyse SEC:

L'appareillage utilisé est un chromatographe « WATERS alliance ». Le solvant d'élution est le mélange suivant : tétrahydrofurane + 1 % vol. de diisopropylamine + 1 % vol. de triéthylamine ou du chloroforme selon le solvant utilisé pour la mise en solution de l'élastomère. Le débit est de 0,7 mL/min, la température du système de 35°C et la durée d'analyse de 90 min. On utilise un jeu de quatre colonnes WATERS en série, de dénominations commerciales «STYRAGEL HMW7», «STYRAGEL HMW6E» et deux «STYRAGEL HT6E».

Le volume injecté de la solution de l'échantillon de l'élastomère est 100 µL. Le détecteur est un réfractomètre différentiel « WATERS 2410 » de longueur d'onde 810 nm. Le logiciel d'exploitation des données chromatographiques est le système «WATERS EM POWER». Les masses molaires moyennes calculées sont relatives à une courbe d'étalonnage réalisée à partir de polystyrènes étalons commerciaux « PSS READY CAL-KIT ».

### 1.3 Caractérisations des molécules

L'analyse structurale ainsi que la détermination des puretés molaires des molécules de synthèse sont réalisées par une analyse RMN. Les spectres sont acquis sur un spectromètre « Avance 3 400 MHz BRUKER » équipé d'une sonde «large bande BBFO-zgrad 5 mm». L'expérience RMN ¹H quantitative utilise une séquence simple impulsion 30° et un délai de répétition de 3 secondes entre chacune des 64 acquisitions. Les échantillons sont solubilisés dans un solvant deutéré, le diméthylsulfoxide deutéré (DMSO) sauf indication contraire. Le solvant deutéré est également utilisé pour le signal de « lock ». Par exemple, la calibration est réalisée sur le signal des protons du DMSO deutéré à 2,44 ppm par rapport à une référence TMS à 0 ppm. Le spectre RMN ¹H couplé aux expériences 2D HSQC ¹H/¹³C et HMBC ¹H/¹³C permettent la détermination structurale des molécules (cf. tableaux d'attributions). Les quantifications molaires sont réalisées à partir du spectre RMN 1D ¹H quantitatif.

L'analyse par spectrométrie de masse est réalisée en injection directe par un mode d'ionisation en électrospray (ID/ESI). Les analyses ont été réalisées sur un spectromètre HCT Bruker (débit 600 µL/min, pression du gaz nébuliseur 10 psi, débit du gaz nébuliseur 4 L/min).

### 1.4 Caractérisations des composés greffés sur les élastomères diéniques

La détermination du taux molaire des composés greffés sur les élastomères diéniques est réalisée par une analyse RMN. Les spectres sont acquis sur un spectromètre « 500 MHz BRUKER » équipé d'une «CryoSonde BBFO-zgrad-5 mm». L'expérience RMN ¹H quantitative, utilise une séquence simple impulsion 30° et un délai de répétition de 5 secondes entre chaque acquisition. Les échantillons sont solubilisés dans le chloroforme deutéré (CDCl₃) dans le but d'obtenir un signal de «lock». Des expériences RMN 2D ont permis de vérifier la nature du motif greffé grâce aux déplacements chimiques des atomes de carbone et de proton.

### 1.5 Propriétés dynamiques

Les propriétés dynamiques G* et tan(δ)max sont mesurées sur un viscoanalyseur (Metravib VA4000), selon la norme ASTM D5992-96. On enregistre la réponse d'un échantillon de composition vulcanisée (éprouvette cylindrique de 4 mm d'épaisseur et de 400 mm² de section), soumis à une sollicitation sinusoïdale en cisaillement simple alterné, à la fréquence de 10 Hz, dans les conditions normales de température (23°C) selon la norme ASTM D1349-09. On effectue un balayage en amplitude de déformation de 0,1 % à 50 % crête-crête (cycle aller) puis de 50 % à 0,1 % crête-crête (cycle retour). Les résultats exploités sont le module complexe de cisaillement dynamique G*à 23°C et le facteur de perte dynamique tan(δ) à 23°C. Pour l'aller, on enregistre la valeur du module complexe de cisaillement dynamique G* à 50 % de déformation, noter G*_{50% aller} à 23°C Pour le retour ; on enregistre la valeur maximale du facteur perte dynamique tan(δ) observée, notée tan(δ)ₘₐₓ à 23°C.

Les résultats sont indiqués en base 100 ; la valeur arbitraire 100 étant attribuée au témoin pour calculer et comparer ensuite tan(δ)ₘₐₓ à 23°C et G*_{50%aller} à 23°C des différents échantillons testés.

Pour tan(δ)ₘₐₓ à 23°C: la valeur en base 100 pour l'échantillon à tester est calculée selon l'opération : (valeur de tan(δ)ₘₐₓ à 23°C de l'échantillon à tester/valeur de tan(δ)ₘₐₓ à 23°C du témoin) × 100. De cette façon, un résultat inférieur à 100 indique une diminution de l'hystérèse qui correspond à une amélioration de la performance de résistance au roulement.

Pour G*_{50% aller} à 23°C : la valeur en base 100 pour l'échantillon à tester est calculée selon l'opération : (valeur de G*_{50% aller} à 23°C de l'échantillon à tester/valeur de G*_{50% aller} à 23°C du témoin) × 100. De cette façon, un résultat supérieur à 100 indique une amélioration du module complexe de cisaillement dynamique G*_{50%aller} de déformation à 23°C, qui corrobore d'une amélioration de la rigidité du matériau.

### 2 Synthèse des composés

### 2.1 - Synthèse du composé A : l'oxyde (2-(2-(3-chloro-2-oxoimidazolidin-1-yl)éthoxy)-1-naphthonitrile

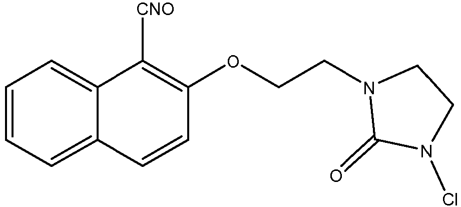

L'oxyde de 2-(2-(3-chloro-2-oxoimidazolidin-1-yl)éthoxy]-1-naphthonitrile est synthétisé en six étapes, nommées étape a1, étape a2, étape b1, étape b2, étape c et étape d.

L'étape a1 est réalisée selon le protocole 1, l'étape a2 selon le protocole 2, l'étape b1 selon le protocole 3. Les étapes b2 et c sont réalisées selon le protocole 4. L'étape d est réalisée selon le protocole 5.

### Étape a1 : préparation du 2-hydroxy-1-naphthaldéhyde

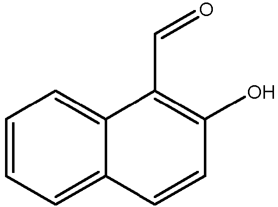

### Protocole 1 :

Ce composé peut être obtenu à partir du naphthol par une réaction de formylation selon le protocole dit de Reimer-Tiemann décrit dans Organic Syntheses, 22, 63-4; 1942 par Russell, Alfred et Lockhart, Luther B. ou dans Organic Reactions (Hoboken, NJ, United States), 28, 1982 par Wynberg, Hans et Meijer, Egbert W., ou selon la procédure décrite par Casiraghi, Giovanni et al. dans Journal of the Chemical Society, Perkin Transactions 1: Organic and Bio-Organic Chemistry (1972-1999), (9), 1862-5, 1980 ou encore par une réaction de Vilsmeier décrite par Jones, Gurnos et Stanforth, Stephen P. dans Organic Reactions (Hoboken, NJ, United States), 49, 1997.

Le 2-hydroxy-1-napthaldéhyde est commercial. Il peut par exemple être obtenu chez Aldrich (CAS 708-06-5).

### Étape a2 : préparation de la 1-(2-chloroéthyl)imidazolidin-2-one

### Protocole 2 :

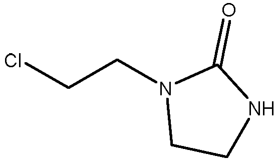

Ce composé peut être obtenu selon un protocole décrit dans la demande de brevet WO 2012/007684.

### Etape b1 : préparation du 2-(2-(2-oxoimidazolidin-1-yl)éthoxy)-1-naphthaldéhyde

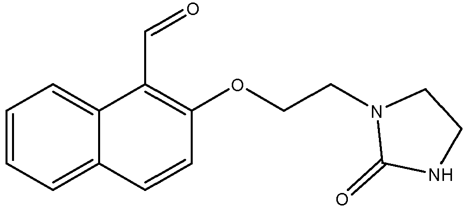

### Protocole 3 :

Un mélange de 2-hydroxy-1-naphthaldéhyde (20,0 g ; 0,116 mol), de carbonate de potassium (24,08 g ; 0,174 mol), et de 1-(2-chloroéthyl)imidazolidin-2-one (25,9 g ; 0,174 mol) dans le DMF (20 mL) est chauffé à 75°C (température du bain) pendant 3,0-3,5 heures. Ensuite, une seconde portion de 1-(2-chloroéthyl)imidazolidin-2-one (17,26 g ; 0,116 mol) est ajoutée et le milieu réactionnel est agité pendant 9 à 10 heures à 75°C (température du bain). Après un retour à 40-45°C, le milieu réactionnel est versé dans une solution d'hydroxyde de sodium (10 g ; 0,25 mol) dans l'eau (700 mL). Le mélange est agité pendant 10 à 15 minutes. Le précipité est filtré et lavé sur le filtre par l'eau distillée (3 fois 400 mL).

Un solide gris (31,51 g ; rendement de 95 %) est obtenu.

La pureté molaire est supérieure à 85 % (RMN ¹H).

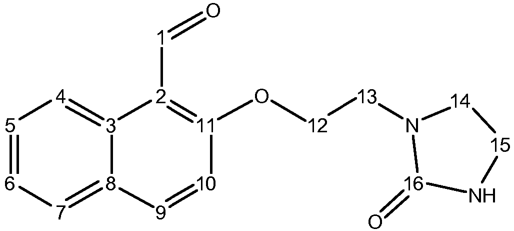

| N° | δ¹H (ppm) | δ¹³C (ppm) |
|---|---|---|
| 1 | 10,86 | 191,6 |
| 2 | / | 116,7 |
| 3 | / | 131,5 |
| 4 | 9,19 | 124,9 |
| 5 | 7,56 | 130 |
| 6 | 7,37 | 124,9 |
| 7 | 7,71 | 128,3 |
| 8 | / | 128,6 |
| 9 | 7,99 | 137,7 |
| 10 | 7,2 | 113,1 |
| 11 | / | 162,9 |
| 12 | 4,31 | 68,7 |
| 13 | 3,63 | 13,4 |
| 14 | 3,59 | 16,8 |
| 15 | 3,72 | 38,3 |
| 16 | / | 162,6 |

### Solvant : CDCl₃

### Étapes b2 et c : préparation de l'oxime de 2-(2-(2-oxoimidazolidin-1-yl)éthoxy)-1-naphthaldéhyde

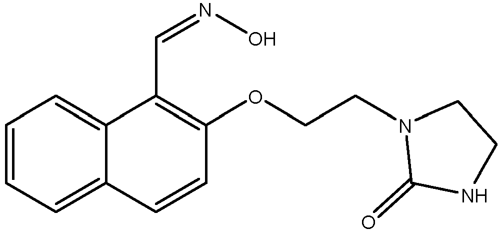

Le produit isolé à l'issue du protocole 3 est utilisé dans le protocole 4 suivant.

### Protocole 4 :

A une solution de 2-(2-(2-oxoimidazolidin-1-yl)éthoxy)-1-naphthaldéhyde (6,3 g ; 22,2 mmol) dans l'éthanol (30 mL) à 45°C (température du bain), est ajoutée une solution d'hydroxylamine (2,05 g ; 31,0 mmol, 50 % dans l'eau, Aldrich) dans l'éthanol (5 mL). Le milieu réactionnel est agité pendant 4 heures à 55 °C (température du bain). Après un retour à 40°C, de l'acétate d'éthyle (30 mL) est ajouté goutte à goutte pendant 15 minutes. Le précipité est filtré, lavé sur le filtre par un mélange éthanol/acétate d'éthyle.

Un solide blanc (4,00 g ; rendement de 60 %) est obtenu.

La pureté molaire estimée par RMN ¹H est supérieure à 95 %.

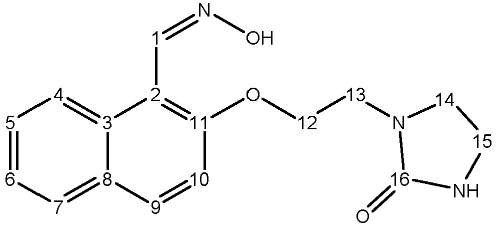

| N° | δ¹H (ppm) | δ¹³C (ppm) |
|---|---|---|
| 1 | 8,75 | 147,5 |
| 2 | / | 115,9 |
| 3 | / | 133 |
| 4 | 8,78 | 127 |
| 5 | 7,46 | 128,5 |
| 6 | 7,35 | 125,2 |
| 7 | 7,88 | 132,8 |
| 8 | / | 130,9 |
| 9 | 7,79 | 129,4 |
| 10 | 7,36 | 115,2 |
| 11 | / | 157,1 |
| 12 | 4,28 | 69,3 |
| 13 | 3,59 | 44,4 |
| 14 | 3,64 | 47,6 |
| 15 | 3,40 | 39,3 |
| 16 | / | 165,3 |

### Solvant MeOH

### Étape d : préparation de l'oxyde de 2-(2-(3-chloro-oxoimidazolidin-1-yl)éthoxy)-1-naphthonitrile

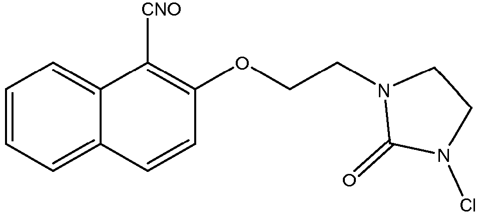

### Protocole 5 :

A une suspension de l'oxime de 2-(2-(2-oxoimidazolidin-1-yl)éthoxy)-1-naphthaldéhyde précédente (7,30 g ; 24,4 mmol) dans l'acétate d'éthyle (230 mL) refroidi à 1°C (Température bain = 0°C) est ajouté au goutte à goutte une solution aqueuse de NaOCl (74,44 g/L ; 98 mL ; 4,0 eq. (eq=équivalent molaire) pendant 10 minutes. Le milieu réactionnel est agité pendant 8,5 heures à 0-2°C (température bain = 0°C).

Une deuxième portion de solution aqueuse de NaOCl (74,44 g/L ; 98 mL ; 4,0 eq.) est ajoutée pendant 5 minutes. Le milieu réactionnel est agité pendant 10-11 heures à 0-2°C (température bain = 0°C).

Le précipité est filtré et lavé sur filtre par l'eau (2 fois 25 mL) et puis par de l'acétate d'éthyle (2 fois 10 mL).

Un solide blanc de point de fusion 127,0-127,5 °C est alors obtenu (6,24 g ; 18,7 mmol, rendement 77 %).

Le composé A, l'oxyde de 2-(2-(3-chloro-2-oxoimidazolidin-1-yl)éthoxy)-1-naphthonitrile, est obtenu avec une pureté molaire est supérieure à 92 % (RMN 1H) et moins de 6 % molaire d'oxyde de 2-(2-(2-oxoimidazolidin-1-yl)éthoxy)-1-naphthonitrile et 2 % molaire d'impureté de solvant résiduel.

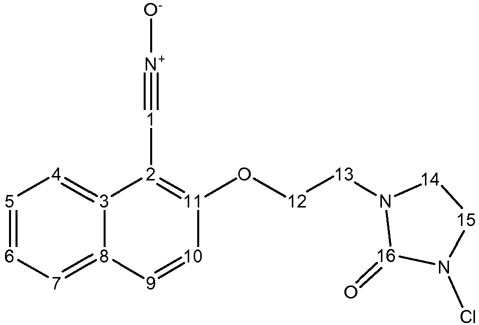

| N° | δ¹H (ppm) | δ¹³C (ppm) |
|---|---|---|
| 1 | / | / |
| 2 | / | 96,9 |
| 3 | / | 134,0 |
| 4 | 7,93 | 124,1 |
| 5 | 7,57 | 129,1 |
| 6 | 7,40 | 125,3 |
| 7 | 7,78 | 128,7 |
| 8 | / | 128,7 |
| 9 | 7,90 | 133,1 |
| 10 | 7,16 | 112,6 |
| 11 | / | 160,2 |
| 12 | 4,33 | 68,8 |
| 13 | 3,71 | 44,8 |
| 14 | 3,75 | 45,2 |
| 15 | 3,58 | 52,1 |
| 16 | / | 161,3 |

### Solvant CDCl₃

### 2.2 - Synthèse du composé B : l'oxyde 2-(2-(2-oxoimidazolidin-1-yl)éthoxy)-1-naphthonitrile

L'oxyde de 2-(2-(2-oxoimidazolidin-1-yl)éthoxy)-1-naphthonitrile est obtenu en reproduisant les étapes des protocoles 1 à 4 du paragraphe 2.1 pour obtenir l'oxime de (2-(2-(2-oxoimidazolidin-1-yl)éthoxy)-1-naphthaldéhyde, puis en effectuant le protocole 6 décrit ci-dessous.

### Protocole 6 :

A une solution d'oxime de 2-(2-(2-oxoimidazolidin-1-yl)éthoxy)-1-naphthaldéhyde (7,5 g ; 25,06 mmol) dans le dichlorométhane (300 mL) refroidi à 4°C (température bain = 0°C), est ajoutée, goutte à goutte, une solution aqueuse de NaOCl (78 g/L ; 35 mL ; 1,46 eq) pendant 15 minutes. Le milieu réactionnel est agité pendant 60 à 70 minutes à une température de 4-5°C. La phase organique est séparée. La phase aqueuse est extraite par le dichlorométhane (25 mL). Les solutions organiques réunies sont lavées par l'eau (2 fois par 25 mL). Le solvant est évaporé sous pression réduite jusqu'à 40-50 mL. De l'éther de pétrole 40/60 (60 mL) est ajouté pour la cristallisation. Le précipité obtenu est filtré et lavé par l'éther de pétrole 40/60 (2 fois par 30 mL).

Un solide blanc (6,46 g ; rendement de 87 %) est obtenu.

Le produit B est obtenu avec une pureté molaire estimée par RMN ¹H est supérieure à 99 % et moins de 1 % d'impuretés de solvant résiduel.

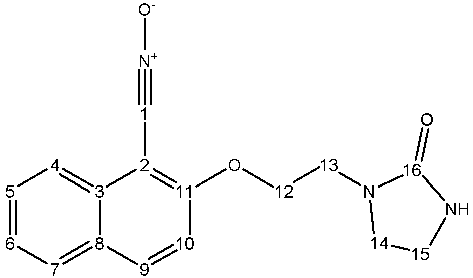

| N° | δ¹H (ppm) | δ¹³C (ppm) |
|---|---|---|
| 1 | / | / |
| 2 | / | 96,7 |
| 3 | / | 134,1 |
| 4 | 7,92 | 124 |
| 5 | 7,56 | 128,9 |
| 6 | 7,39 | 125,2 |
| 7 | 7,77 | 128,6 |
| 8 | / | 128,5 |
| 9 | 7,89 | 133 |
| 10 | 7,18 | 112,7 |
| 11 | / | 160,5 |
| 12 | 4,31 | 69,4 |
| 13 | 3,64 | 43,4 |
| 14 | 3,76 | 47,3 |
| 15 | 3,42 | 38,5 |
| 16 | / | 162,5 |

### Solvant : CDCl₃

### 3 Exemple de polymères modifiés

### 3.1 Copolymère styrène-butadiène greffé par de l'oxyde de 2-(2-(3-chloro-2-oxoimidazolidin-1-yl)éthoxy)-1-naphthonitrile

On incorpore l'oxyde de 2-(2-(3-chloro-2-oxoimidazolidin-1-yl)éthoxy)-1-naphthonitrile (0,27 g; 0,81 mmol), composé A obtenu par le procédé de synthèse décrit au paragraphe 2.1, à 20 g de copolymère styrène-butadiène non-fonctionnalisé (contenant 26,5 % en poids de styrène par rapport au poids total du polymère et 25 % en poids d'unité butadiène-1,2, par rapport au poids de la partie butadiènique ; Mn = 150000 g/mol et Ip=1,84 mesurés selon la méthode décrite au paragraphe 1.2) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

L'analyse par RMN ¹H a permis de démontrer un taux molaire de greffage de 0,29 % avec un rendement molaire de greffage de 96 %.

Déplacement chimique permettant la détermination du taux de greffage :
7.6 à 8.2 ppm : 6 H aromatiques
4.2 4.3 ppm : 2H CH₂-O
3.1-3.8 ppm : 6H CH₂-N

### 3.2 Polyisoprène greffé par de l'oxyde de 2-(2-(3-chloro-2-oxoimidazolidin-1-yl)éthoxy)-1-naphthonitrile

On incorpore l'oxyde de 2-(2-(3-chloro-2-oxoimidazolidin-1-yl)éthoxy)-1-naphthonitrile (0,29 g; 0,88 mmol), composé A obtenu par le procédé de synthèse décrit au paragraphe 1.2, à 20 g d'un polyisoprène synthétique (contenant 99,35% en poids d'unité isoprène-1,4 cis et 0,65 % en poids d'unité isoprène 3,4 ; Mn=375000 g/mol et Ip=3,6 mesurés selon la méthode décrite au paragraphe 1.2) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

L'analyse par RMN ¹H a permis de démontrer un taux molaire de greffage de 0,2 % avec un rendement molaire de greffage de 66 %.

Déplacement chimique permettant la détermination du taux de greffage :
7.6 à 8.2 ppm : 6 H aromatiques
4.2 4.3 ppm : 2H CH₂-O
3.1-3.8 ppm : 6H CH₂-N

### 4 Exemple de compositions de caoutchouc

### 4.1 Exemple 1

Cet essai a pour objet de montrer l'amélioration de performance en terme de propriétés de caoutchouterie d'une composition de l'invention par rapport à une composition non conforme classique ne contenant pas d'élastomère modifié et à une composition de l'art antérieur comprenant un élastomère modifié. En particulier, ces compositions sont définies de la façon suivante :
- la composition non-conforme C1 est une composition « classique » de caoutchouc utilisée comme bande de roulement pour pneumatique, incluant notamment un polyisoprène de synthèse non greffé, de la silice et un agent de couplage polysulfure silane ;
- la composition non conforme C2 est identique à la composition C1 à l'exception qu'elle comprend en plus le composé B qui se greffe sur le polyisoprène de synthèse ;
- la composition C3, conforme à l'invention, est une composition identique à la composition C1 à l'exception qu'elle comprend en plus le composé A qui se greffe sur le polyisoprène de synthèse.

Le taux des différents constituants de ces compositions exprimés en pce, partie en poids pour cent parties en poids d'élastomère, sont présentés dans le tableau 1:

**Tableau 1**

| | **C1** | **C2** | **C3** |
|---|---|---|---|
| Élastomère diénique (1) | 100 | 100 | 100 |
| Composé B (2) | | 1,31 | |
| Composé A (3) | | | 1,46 |
| Charge renforçante (4) | 60 | 60 | 60 |
| Agent de couplage (5) | 6 | 6 | 6 |
| Noir de carbone (6) | 3 | 3 | 3 |
| Antioxydant (7) | 1,5 | 1,5 | 1,5 |
| TMQ (8) | 1 | 1 | 1 |
| Paraffine | 1 | 1 | 1 |
| ZnO (9) | 2,7 | 2,7 | 2,7 |
| Acide stéarique (10) | 2,5 | 2,5 | 2,5 |
| CBS (11) | 1,6 | 1,6 | 1,6 |
| Soufre | 1,3 | 1,3 | 1,3 |

Les compositions C2 et C3 comprennent le même nombre de mol de composé A ou B greffé, à savoir 0,2 % molaire.
(1) Polyisoprène contenant 99,35% en poids d'unité isoprène-1,4 cis et 0,65 % en poids d'unité isoprène 3,4 ; Mn=375000 g/mol et Ip=3,6 mesurés selon la méthode décrite au paragraphe 1.2 ;
(2) Composé B : oxyde de 2-(2-(2-oxoimidazolidin-1-yl)éthoxy)-1-naphthonitrile synthétisé selon le procédé décrit au paragraphe 2.2 ;
(3) Composé A : oxyde de 2-(2-(3-chloro-2-oxoimidazolidin-1-yl)éthoxy)-1-naphthonitrile synthétisé selon le procédé décrit au paragraphe 2.1 ;
(4) Silice « Zeosil 1165MP » commercialisée par Solvay ;
(5) Bis[3-(triéthoxysilyl)propyl] tétrasulfure silane (TESPT) commercialisé par Evonik sous la référence « Si69 » ;
(6) Noir de carbone de grade N234 commercialisé par Cabot Corporation ;
(7) N-1,3-diméthylbutyl-N-phenyl-para-phénylènediamine commercialisé par Flexys sous la référence « Santoflex 6-PPD » ;
(8) 2,2,4-triméthyl-1,2-dihydroquinoline commercialisée par la société Flexys ;
(9) Oxyde de Zinc (grade industriel) commercialisé par la société Umicore ;
(10) Stéarine « Pristerene 4031 » commercialisée par la société Uniquema ;
(11) N-cyclohexyl-2-benzothiazyl-sulfénamide commercialisé par Flexys sous la référence « Santocure CBS ».

Les compositions C1 à C3 sont préparées de la manière suivante : on introduit dans un mélangeur interne « Polylab » de 85 cm³, rempli à 70 % et dont la température initiale de cuve est d'environ 110°C, l'élastomère diénique. Pour les compositions concernant le composé A ou le composé B, le composé A ou le composé B est introduit en même temps que l'élastomère diénique. Puis un travail thermomécanique d'une minute et demie est conduit à une température de 120°C.

Ensuite, pour chacune des compositions C1 à C3, on introduit la ou les charges renforçantes, l'agent de couplage de la charge avec l'élastomère diénique, puis après une à deux minutes de malaxage, les divers autres ingrédients à l'exception du système de vulcanisation. On conduit alors un travail thermomécanique (phase non-productive) en une étape, qui dure au total environ 5 minutes, jusqu'à atteindre une température maximale de tombée de 160°C.

On récupère le mélange ainsi obtenu, on le refroidit puis on ajoute le système de vulcanisation (soufre et l'accélérateur type sulfénamide) sur un mélangeur externe (homo-finisseur) à 25°C, en mélangeant l'ensemble (phase productive) pendant environ 5 à 6 minutes.

Les compositions ainsi obtenues sont ensuite calandrées sous la forme de plaques (épaisseur de 2 à 3 mm) ou de feuilles fines de caoutchouc pour la mesure de leurs propriétés physiques ou mécaniques.

Les propriétés de caoutchouterie de ces compositions sont mesurées après cuisson à 150°C pendant 60 minutes. Les résultats obtenus sont portés dans le tableau 2.

**Tableau 2**

| Compositions | **C1** | **C2** | **C3** |
|---|---|---|---|
| Tan(δ)ₘₐₓ à 23 C° | 100 | 80 | 70 |

On constate au vu du tableau 2, de façon attendue, que la composition non conforme C2 qui comprend un élastomère modifié avec un composé comportant une fonction imidazolidinone non substituée, présente une diminution de l'hystérèse (tan(δ)ₘₐₓ à 23°C), par rapport à la composition non conforme C1 qui ne comprend pas d'élastomère modifié.

De façon surprenante, la composition C3 selon l'invention qui comprend un élastomère modifié avec un composé comportant une fonction imidazolidinone N-substituée présente une diminution significative de l'hystérèse par rapport à la composition non conforme C1 ainsi qu'une diminution significative de l'hystérèse par rapport à la composition non conforme C2 qui présentait déjà de bonnes propriétés hystérétiques par rapport à la composition non conforme C1.

L'élastomère modifié selon l'invention permet donc de diminuer l'hystérèse des compositions le contenant, et donc d'améliorer la performance résistance au roulement de ces compositions.

### 4.2 Exemple 2

Cet essai a pour objet de montrer l'amélioration des propriétés de caoutchouterie de plusieurs compositions selon l'invention par rapport à des compositions non conformes classiques ne comprenant pas d'élastomère modifié et à des compositions de l'art antérieur non conformes comprenant un élastomère modifié. En particulier, ces compositions sont définies de la façon suivante :
- la composition non-conforme C4 est une composition « classique » de caoutchouc utilisée comme bande de roulement pour pneumatique, incluant notamment un copolymère styrène-butadiène (SBR) non greffé, de la silice et un agent de couplage polysulfure silane ;
- la composition non conforme C5 est identique à la composition C4 à l'exception qu'elle comprend en plus le composé B qui se greffe sur le SBR ;
- la composition C6, conforme à l'invention, est une composition identique à la composition C5 à l'exception qu'elle comprend en plus les composés A et B qui se greffent sur le SBR ;
- la composition non-conforme C7 est identique à la composition C4 sauf qu'elle comprend un polyisoprène de synthèse à la place du SBR ;
- la composition non conforme C8 est identique à la composition C7 à l'exception qu'elle comprend en plus le composé B qui se greffe sur le polyisoprène de synthèse ;
- la composition C9, conforme à l'invention, est une composition identique à la composition C7 à l'exception qu'elle comprend en plus les composés A et B qui se greffent sur le polyisoprène de synthèse.

Le taux des différents constituants des compositions exprimés en pce, partie en poids pour cent parties en poids d'élastomère, sont présentés dans le tableau 3 qui suit :

**Tableau 3**

| | **C4** | **C5** | **C6** | **C7** | **C8** | **C9** |
|---|---|---|---|---|---|---|
| Élastomère diénique (1) | | | | 100 | 100 | 100 |
| Élastomère diénique (2) | 100 | 100 | 100 | | | |
| Composé B (3) | | 1,31 | 1,31 | | 1,31 | 1,31 |
| Composé A (4) | | | 0,49 | | | 0,49 |
| Charge renforçante (5) | 60 | 60 | 60 | 60 | 60 | 60 |
| Agent de couplage (6) | 6 | 6 | 6 | 6 | 6 | 6 |
| Noir de carbone (7) | 3 | 3 | 3 | 3 | 3 | 3 |
| Antioxydant (8) | 1,5 | 1,5 | 1,5 | 1,5 | 1,5 | 1,5 |
| TMQ (9) | 1 | 1 | 1 | 1 | 1 | 1 |
| Paraffine | 1 | 1 | 1 | 1 | 1 | 1 |
| ZnO (10) | 2,7 | 2,7 | 2,7 | 2,7 | 2,7 | 2,7 |
| Acide stéarique (11) | 2,5 | 2,5 | 2,5 | 2,5 | 2,5 | 2,5 |
| CBS (12) | 1,6 | 1,6 | 1,6 | 1,6 | 1,6 | 1,6 |
| Soufre | 1,3 | 1,3 | 1,3 | 1,3 | 1,3 | 1,3 |

| | | | | | | |
|---|---|---|---|---|---|---|
| (1) Polyisoprène contenant 99,35% en poids d'unité isoprène-1,4 cis et 0,65 % en poids d'unité isoprène 3,4 ; Mn=375000 g/mol et Ip=3,6 mesurés selon la méthode décrite au paragraphe 1.2 ; (2) Copolymère de styrène-butadiène non-fonctionnalisé contenant 26,5% en poids de styrène par rapport au poids total du copolymère et 25 % en poids d'unité butadiène-1,2 par rapport au poids de la partie butadiènique ; Mn = 150000 g/mol et Ip=1,84 mesurés selon la méthode décrite au paragraphe 1.2 ; (3) Composé B : oxyde de 2-(2-(2-oxoimidazolidin-1-yl)éthoxy)-1-naphthonitrile synthétisé selon le procédé décrit au paragraphe 2.2 ; (4) Composé A : oxyde de 2-(2-(3-chloro-2-oxoimidazolidin-1-yl)éthoxy)-1-naphthonitrile synthétisé selon le procédé décrit au paragraphe 2.1 ; (5) Silice « Zeosil 1165MP » commercialisée par Solvay ; (6) Bis[3-(triéthoxysilyl)propyl] tétrasulfure silane (TESPT) commercialisé par Evonik sous la référence « Si69 » ; (7) Noir de carbone de grade N234 commercialisé par Cabot Corporation ; (8) N-1,3-diméthylbutyl-N-phenyl-para-phénylènediamine commercialisé par Flexys sous la référence « Santoflex 6-PPD » ; (9) 2,2,4-triméthyl-1,2-dihydroquinoline commercialisée par la société Flexys ; (10) Oxyde de Zinc (grade industriel) commercialisé par la société Umicore ; (11) Stéarine « Pristerene 4031 » commercialisée par la société Uniquema ; (12) N-cyclohexyl-2-benzothiazyl-sulfénamide commercialisé par Flexys sous la référence « Santocure CBS ». | | | | | | |

Les compositions sont préparées de la manière suivante : on introduit dans un mélangeur interne « Polylab » de 85 cm³, rempli à 70% et dont la température initiale de cuve est d'environ 110°C, l'élastomère diénique. Pour les compositions concernant le composé B ou les composés A et B, le composé B ou les composés A et B sont introduits en même temps que l'élastomère diénique. Puis un travail thermomécanique d'une minute et demie est conduit à une température de 120°C.

Ensuite, pour chacune des compositions C4 à C9, on introduit la ou les charges renforçantes, l'agent de coupage de la charge avec l'élastomère diénique, puis après une à deux minutes de malaxage, les divers autres ingrédients à l'exception du système de vulcanisation. On conduit alors un travail thermomécanique (phase non-productive) en une étape, qui dure au total environ 5 minutes, jusqu'à atteindre une température maximale de tombée de 160°C.

On récupère le mélange ainsi obtenu, on le refroidit puis on ajoute le système de vulcanisation (soufre et l'accélérateur type sulfénamide) sur un mélangeur externe (homo-finisseur) à 25°C, en mélangeant l'ensemble (phase productive) pendant environs 5 à 6 minutes.

Les compositions ainsi obtenues sont ensuite calandrées sous la forme de plaques (épaisseur de 2 à 3 mm) ou de feuilles fines de caoutchouc pour la mesure de leurs propriétés physiques ou mécaniques. Les propriétés de caoutchouterie de ces compositions sont mesurées après cuisson à 150°C pendant 60 min. Les résultats obtenus sont portés dans le tableau 4.

**Tableau 4**

| | **C4** | **C5** | **C6** | **C7** | **C8** | **C9** |
|---|---|---|---|---|---|---|
| G *_{50% aller} à 23°C | 100 | 100 | 99 | 100 | 100 | 93 |
| Tan (δ) ₘₐₓ à 23 C° | 100 | 81 | 76 | 100 | 80 | 65 |

On constate, au vu du tableau 4, de façon attendue, que les compositions non conforme C5 et C8 qui comprennent un élastomère greffé avec un composé comportant des fonctions imidazolidinone non-substituées, présentent une diminution de l'hystérèse à iso-rigidité (G *_{50% aller} à 23°C) par rapport aux compositions non conformes C4 et C7 respectivement qui ne comprennent pas d'élastomère modifié.

De façon surprenante, la composition C6 selon l'invention qui comprend un élastomère modifié avec un composé portant des fonctions imidazolidinone N-substituées et un composé portant des fonctions imidazolidinone non-substituées présente une diminution de l'hystérèse significative par rapport à la composition non conforme C5 qui présentait déjà une hystérèse diminuée par rapport à la composition non conforme C4. Cette diminution de l'hystérèse s'effectue sans une diminution des propriétés de rigidité à cuit, ce qui est surprenant. En effet, l'homme du métier sait que l'hystérèse et les propriétés de rigidité à cuit sont deux propriétés antagonistes des compositions de caoutchouc. Il est connu qu'une amélioration des propriétés de rigidité à cru entraîne une dégradation de l'hystérèse et inversement. Or on observe ici une amélioration significative du compromis rigidité à cuit/hystérèse de la composition C6 conforme à l'invention, par rapport aux compositions C4 et C5 non conformes. Ce même résultat est obtenu avec la composition C9 conforme à l'invention par rapport aux compositions non conformes C7 et C8 ; même si une baisse de la rigidité à cru est observée pour la composition C9 selon l'invention par rapport à la composition C8 non conforme. Cette diminution de la rigidité à cru reste très faible compte tenu de la forte baisse de l'hystérèse de la composition C9 selon l'invention.

## Revendications

1. Polymère modifié obtenu par greffage d'au moins un composé de formule (I) sur au moins une insaturation de la chaîne d'un polymère initial
dans laquelle : 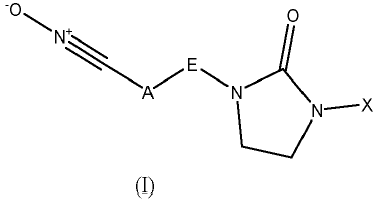
• A représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ;
• E représente un groupe divalent hydrocarboné comprenant éventuellement un ou plusieurs hétéroatomes ; et
• X désigne un atome d'halogène, de préférence un atome de chlore, le polymère initial étant un élastomère diénique.

2. Polymère modifié selon la revendication 1, dans lequel le polymère initial est choisi dans le groupe constitué par les copolymères d'éthylène-propylène-monomère diène, le caoutchouc butyle, le caoutchouc naturel, les polyisoprènes de synthèse, les polybutadiènes, les copolymères de butadiène, les copolymères d'isoprène et les mélanges de ces élastomères.

3. Polymère modifié selon l'une quelconque des revendications précédentes, dans lequel le groupement A est un cycle arènediyle en C6-C14 éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes.

4. Polymère modifié selon l'une quelconque des revendications 1 à 3, dans lequel le composé de formule (I) est choisi parmi les composés de formule (Ia) et (Ib)
dans lesquelles : 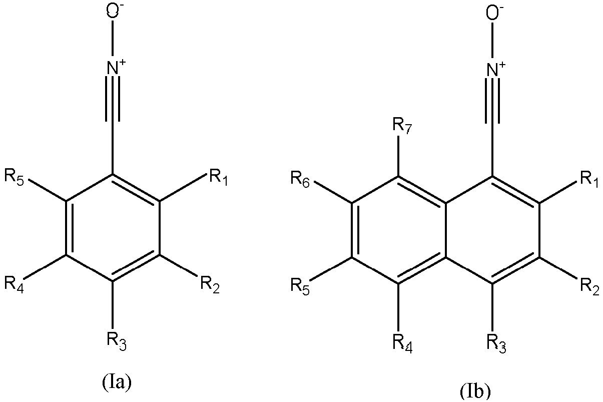
• un groupement choisi parmi R1 à R5 de la formule (Ia) et un groupement choisi parmi R1 à R7 de la formule (Ib) désignent le groupe de formule (II) suivante : 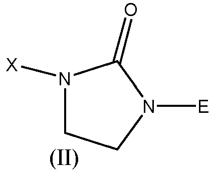 dans laquelle E et X sont tels que défini à la revendication 1 ; et
• les quatre groupements de la formule (Ia) choisis parmi R1 à R5 autres que celui désignant le groupe de formule (II) et les six groupements de la formule (Ib) choisis parmi R1 à R7 autres que celui désignant le groupe de formule (II), identiques ou différents, représentent indépendamment les uns des autres, un atome d'hydrogène ou une chaîne hydrocarbonée aliphatique, de préférence saturée, linéaire ou ramifiée, éventuellement substituée ou interrompue par un ou plusieurs hétéroatomes.

5. Polymère modifié selon l'une quelconque des revendications précédentes, dans lequel le groupement E est une chaîne hydrocarbonée, linéaire ou ramifiée, de préférence saturée, en C1-C24, de préférence en C1-C10, plus préférentiellement en C1-C6 éventuellement interrompue par un ou plusieurs atomes d'azote, de soufre ou d'oxygène.

6. Polymère modifié selon l'une quelconque des revendications 4 à 5, dans lequel le composé de formule (I) est le composé de formule (Ib).

7. Polymère modifié selon l'une quelconque des revendications précédentes, dans lequel le composé de formule (I) est le composé de formule (III) suivante :

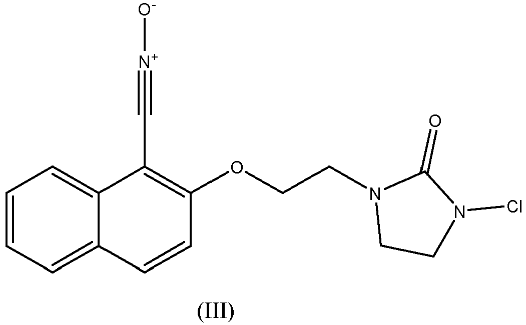

8. Polymère modifié selon l'une quelconque des revendications précédentes, **caractérisé en ce qu'**il est en outre obtenu par greffage d'au moins un composé de formule (V) sur au moins une autre insaturation de la chaîne dudit polymère initial 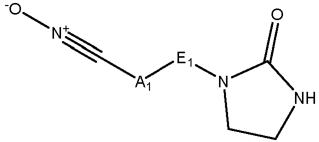 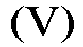 dans laquelle :
• A1 représente un cycle arènediyle, éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ; et
• E1 représente un groupe divalent hydrocarboné comprenant éventuellement un ou plusieurs hétéroatomes.

9. Polymère modifié selon la revendication 8, dans lequel le groupement A1 du composé de formule (V) est un cycle arènediyle en C6-C14 éventuellement substitué par une ou plusieurs chaînes hydrocarbonées, identiques ou différentes, aliphatiques, de préférence, linéaires ou ramifiées, éventuellement substituées ou interrompues par un ou plusieurs hétéroatomes.

10. Polymère modifié selon l'une quelconque des revendications 8 à 9, dans lequel le composé de formule (V) est choisi parmi les composés de formule (Va) et (Vb) 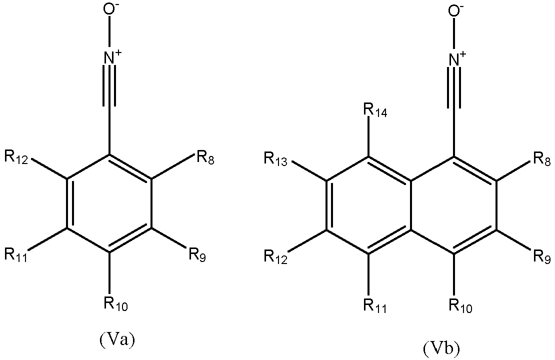 dans lesquelles :
• un groupement choisi parmi R8 à R12 de la formule (Va) et un groupement choisi parmi R8 à R14 de la formule (Vb) désignent le groupe de formule (VI) suivante : 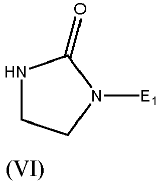 dans laquelle E1 tel que défini à la revendication 8 ; et
• les quatre groupements de la formule (Va) choisis parmi R8 à R12 autres que celui désignant le groupe de formule (VI) et les six groupements de la formule (Vb) choisis parmi R8 à R14 autres que celui désignant le groupe de formule (VI), identiques ou différents, représentent indépendamment les uns des autres, un atome d'hydrogène ou une chaîne hydrocarbonée aliphatique, de préférence saturée, linéaire ou ramifiée, éventuellement substituée ou interrompue par un ou plusieurs hétéroatomes.

11. Polymère modifié selon l'une quelconque des revendications 8 à 10, dans lequel le groupement E1 du composé de formule (V) est une chaîne hydrocarbonée, linéaire ou ramifiée, de préférence saturée, en C1-C24, de préférence en C1-C10, plus préférentiellement en C1-C6 éventuellement interrompue par un ou plusieurs atomes d'azote, de soufre ou d'oxygène.

12. Polymère modifié selon l'une quelconque des revendications 10 à 11, dans lequel le composé de formule (V) est le composé de formule (Vb).

13. Polymère modifié selon l'une quelconque des revendications 8 à 12, dans lequel le composé de formule (V) est le composé de formule (VII) suivante :

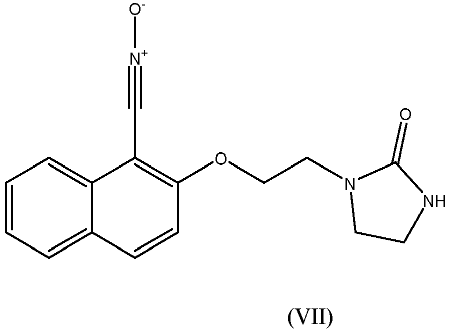

14. Composition comprenant au moins un élastomère diénique modifié tel que défini à l'une quelconque des revendications 1 à 13 et au moins un additif.

## Patentansprüche

1. Modifiziertes Polymer, erhalten durch Pfropfen mindestens einer Verbindung der Formel (I) auf mindestens eine Ungesättigtheit der Kette eines Ausgangspolymers 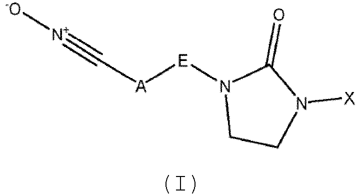 wobei:
• A für einen Arendiylring steht, der gegebenenfalls durch eine oder mehrere gleiche oder verschiedene, vorzugsweise gesättigte, lineare oder verzweigte aliphatische Kohlenwasserstoffketten substituiert ist, die gegebenenfalls durch ein oder mehrere Heteroatome substituiert oder unterbrochen sind;
• E für eine zweiwertige Kohlenwasserstoffgruppe steht, die gegebenenfalls ein oder mehrere Heteroatome umfasst; und
• X ein Halogenatom, vorzugsweise ein Chloratom, bedeutet, wobei es sich bei dem Ausgangspolymer um ein Dienelastomer handelt.

2. Modifiziertes Polymer nach Anspruch 1, wobei das Ausgangspolymer aus der Gruppe bestehend aus Ethylen-Propylen-Dienmonomer-Copolymeren, Butylkautschuk, Naturkautschuk, synthetischen Polyisoprenen, Polybutadienen, Butadien-Copolymeren, Isopren-Copolymeren und Mischungen dieser Elastomere ausgewählt ist.

3. Modifiziertes Polymer nach einem der vorhergehenden Ansprüche, wobei es sich bei der Gruppe A um einen C6-C14-Arendiylring handelt, der gegebenenfalls durch eine oder mehrere gleiche oder verschiedene, vorzugsweise gesättigte, lineare oder verzweigte aliphatische Kohlenwasserstoffketten substituiert ist, die gegebenenfalls durch ein oder mehrere Heteroatome substituiert oder unterbrochen sind.

4. Modifiziertes Polymer nach einem der Ansprüche 1 bis 3, wobei die Verbindung der Formel (I) aus Verbindungen der Formel (Ia) und (Ib) ausgewählt ist: 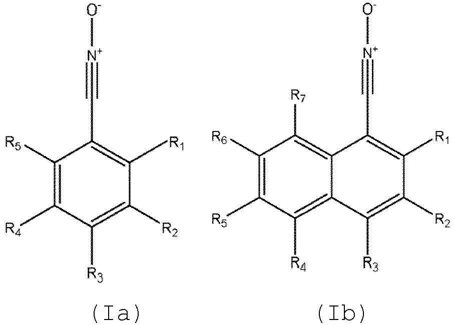 wobei:
• eine Gruppe, die aus R1 bis R5 der Formel (Ia) ausgewählt ist, und eine Gruppe, die aus R1 bis R7 der Formel (Ib) ausgewählt ist, die Gruppe der folgenden Formel (II) bedeuten: 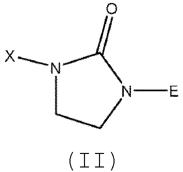 wobei E und X wie in Anspruch 1 definiert sind; und
• die vier Gruppen der Formel (Ia), die aus R1 bis R5 ausgewählt sind, außer derjenigen Gruppe, die die Gruppe der Formel (II) bedeutet, und die sechs Gruppen der Formel (Ib), die aus R1 bis R7 ausgewählt sind, außer derjenigen Gruppe, die die Gruppe der Formel (II) bedeutet, die gleich oder verschieden sind, unabhängig voneinander für ein Wasserstoffatom oder eine vorzugsweise gesättigte, lineare oder verzweigte aliphatische Kohlenwasserstoffkette, die gegebenenfalls durch ein oder mehrere Heteroatome substituiert oder unterbrochen ist, stehen.

5. Modifiziertes Polymer nach einem der vorhergehenden Ansprüche, wobei es sich bei der Gruppe E um eine vorzugsweise gesättigte, lineare oder verzweigte C1-C24-, vorzugsweise C1-C10- und weiter bevorzugt C1-C6-Kohlenwasserstoffkette, die gegebenenfalls durch ein oder mehrere Stickstoff-, Schwefel- oder Sauerstoffatome unterbrochen ist, handelt.

6. Modifiziertes Polymer nach einem der Ansprüche 4 bis 5, wobei es sich bei der Verbindung der Formel (I) um die Verbindung der Formel (Ib) handelt.

7. Modifiziertes Polymer nach einem der vorhergehenden Ansprüche, wobei es sich bei der Verbindung der Formel (I) um die Verbindung der folgenden Formel (III) handelt:

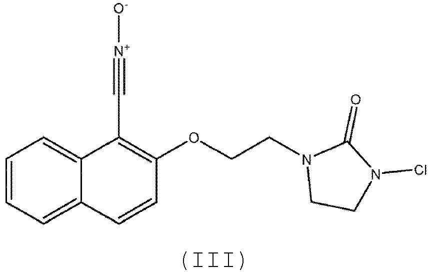

8. Modifiziertes Polymer nach einem der vorhergehenden Ansprüche, **dadurch gekennzeichnet, dass** es außerdem durch Pfropfen mindestens einer Verbindung der Formel (V) auf mindestens eine weitere Ungesättigtheit der Kette des Ausgangspolymers erhalten wird: 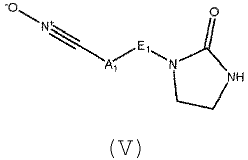 wobei:
• A1 für einen Arendiylring steht, der gegebenenfalls durch eine oder mehrere gleiche oder verschiedene, vorzugsweise gesättigte, lineare oder verzweigte aliphatische Kohlenwasserstoffketten substituiert ist, die gegebenenfalls durch ein oder mehrere Heteroatome substituiert oder unterbrochen sind; und
• E1 für eine zweiwertige Kohlenwasserstoffgruppe steht, die gegebenenfalls ein oder mehrere Heteroatome umfasst.

9. Modifiziertes Polymer nach Anspruch 8, wobei es sich bei der Gruppe A1 der Verbindung der Formel (V) um einen C6-C14-Arendiylring handelt, der gegebenenfalls durch eine oder mehrere gleiche oder verschiedene, vorzugsweise, lineare oder verzweigte aliphatische Kohlenwasserstoffketten substituiert ist, die gegebenenfalls durch ein oder mehrere Heteroatome substituiert oder unterbrochen sind.

10. Modifiziertes Polymer nach einem der Ansprüche 8 bis 9, wobei die Verbindung der Formel (V) aus den Verbindungen der Formel (Va) und (Vb) ausgewählt ist: 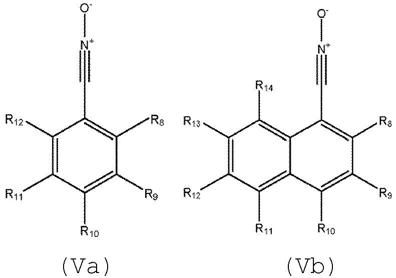 wobei:
• eine Gruppe, die aus R8 bis R12 der Formel (Va) ausgewählt ist, und eine Gruppe, die aus R8 bis R14 der Formel (Vb) ausgewählt ist, die Gruppe der folgenden Formel (VI) bedeuten: 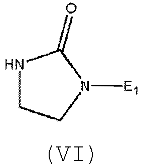 wobei E1 wie in Anspruch 8 definiert sind; und
• die vier Gruppen der Formel (Va), die aus R8 bis R12 ausgewählt sind, außer derjenigen Gruppe, die die Gruppe der Formel (VI) bedeutet, und die sechs Gruppen der Formel (Vb), die aus R8 bis R14 ausgewählt sind, außer derjenigen Gruppe, die die Gruppe der Formel (VI) bedeutet, die gleich oder verschieden sind, unabhängig voneinander für ein Wasserstoffatom oder eine vorzugsweise gesättigte, lineare oder verzweigte aliphatische Kohlenwasserstoffkette, die gegebenenfalls durch ein oder mehrere Heteroatome substituiert oder unterbrochen ist, stehen.

11. Modifiziertes Polymer nach einem der Ansprüche 8 bis 10, wobei es sich bei der Gruppe E1 der Verbindung der Formel (V) um eine vorzugsweise gesättigte, lineare oder verzweigte C1-C24-, vorzugsweise C1-C10- und weiter bevorzugt C1-C6-Kohlenwasserstoffkette, die gegebenenfalls durch ein oder mehrere Stickstoff-, Schwefel- oder Sauerstoffatome unterbrochen ist, handelt.

12. Modifiziertes Polymer nach einem der Ansprüche 10 bis 11, wobei es sich bei der Verbindung der Formel (V) um die Verbindung der Formel (Vb) handelt.

13. Modifiziertes Polymer nach einem der Ansprüche 8 bis 12, wobei es sich bei der Verbindung der Formel (V) um die Verbindung der folgenden Formel (VII) handelt:

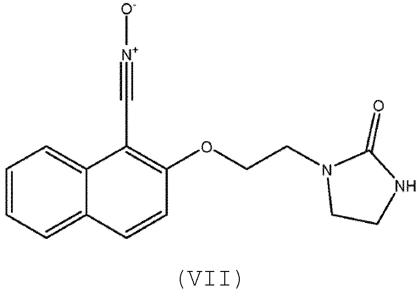

14. Zusammensetzung, umfassend mindestens ein modifiziertes Dienelastomer gemäß einem der Ansprüche 1 bis 13 und mindestens ein Additiv.

## Claims

1. Modified polymer obtained by grafting at least one compound of formula (I) to at least one unsaturation of the chain of an initial polymer:
in which: 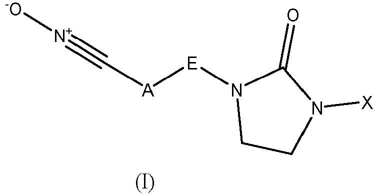
• A represents an arenediyl ring optionally substituted by one or more identical or different, preferably saturated, linear or branched, aliphatic hydrocarbon chains, which are optionally substituted or interrupted by one or more heteroatoms;
• E represents a divalent hydrocarbon group optionally comprising one or more heteroatoms; and
• X denotes a halogen atom, preferably a chlorine atom,
the initial polymer is a diene elastomer.

2. Modified polymer according to Claim 1, in which the initial polymer is selected from the group consisting of ethylene/propylene/diene monomer copolymers, butyl rubber, natural rubber, synthetic polyisoprenes, polybutadienes, butadiene copolymers, isoprene copolymers and the mixtures of these elastomers.

3. Modified polymer according to any one of the preceding claims, in which the group A is a C₆-C₁₄ arenediyl ring optionally substituted by one or more identical or different, preferably saturated, linear or branched aliphatic hydrocarbon chains, which are optionally substituted or interrupted by one or more heteroatoms.

4. Modified polymer according to any one of Claims 1 to 3, in which the compound of formula (I) is chosen from the compounds of formula (Ia) and (Ib): 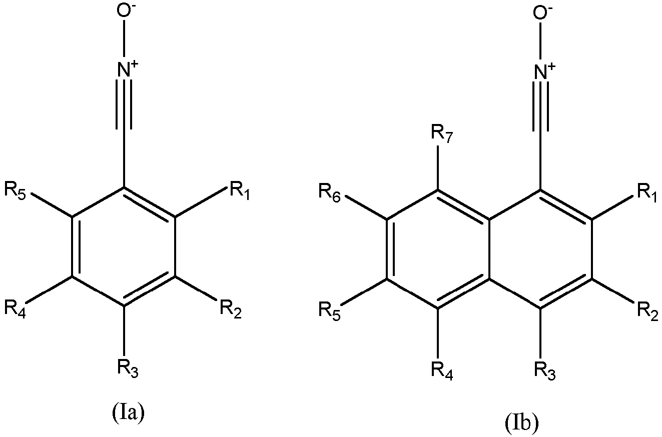 in which:
• a group chosen from R₁ to R₅ of the formula (Ia) and a group chosen from R₁ to R₇ of the formula (Ib) denote the group of following formula (II): 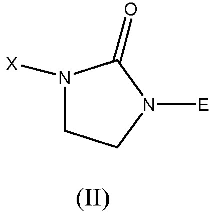 in which E and X are as defined in Claim 1; and
• the four groups of the formula (Ia) chosen from R₁ to R₅ other than that denoting the group of formula (II) and the six groups of the formula (Ib) chosen from R₁ to R₇ other than that denoting the group of formula (II), which are identical or different, represent, independently of one another, a hydrogen atom or a preferably saturated, linear or branched aliphatic hydrocarbon chain optionally substituted or interrupted by one or more heteroatoms.

5. Modified polymer according to any one of the preceding claims, in which the group E is a preferably saturated, linear or branched C₁-C₂₄, preferably C₁-C₁₀, more preferentially C₁-C₆, hydrocarbon chain optionally interrupted by one or more nitrogen, sulfur or oxygen atoms.

6. Modified polymer according to either one of Claims 4 and 5, in which the compound of formula (I) is the compound of formula (Ib).

7. Modified polymer according to any one of the preceding claims, in which the compound of formula (I) is the compound of following formula (III):

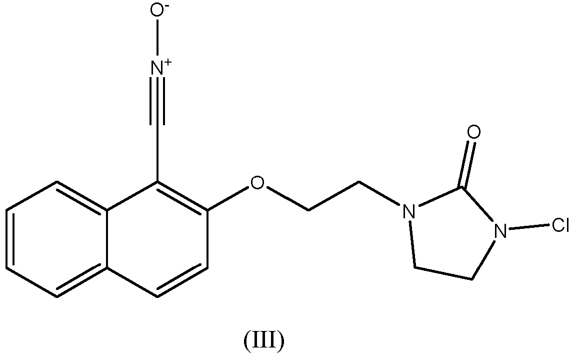

8. Modified polymer according to any one of the preceding claims, **characterized in that** it is additionally obtained by grafting at least one compound of formula (V) to at least one other unsaturation of the chain of said initial polymer: 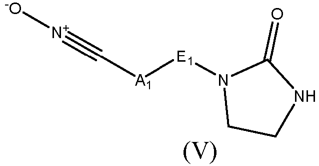 in which:
• A₁ represents an arenediyl ring optionally substituted by one or more identical or different, preferably saturated, linear or branched aliphatic hydrocarbon chains, which are optionally substituted or interrupted by one or more heteroatoms; and
• E₁ represents a divalent hydrocarbon group optionally comprising one or more heteroatoms.

9. Modified polymer according to Claim 8, in which the group A₁ of the compound of formula (V) is a C₆-C₁₄ arenediyl ring optionally substituted by one or more identical or different, preferably, linear or branched aliphatic hydrocarbon chains, which are optionally substituted or interrupted by one or more heteroatoms.

10. Modified polymer according to either one of Claims 8 and 9, in which the compound of formula (V) is chosen from the compounds of formula (Va) and (Vb): 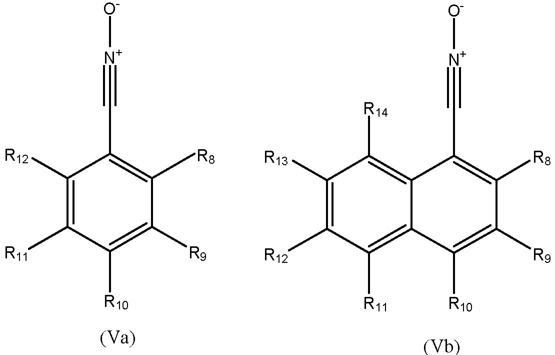 in which:
• a group chosen from R₈ to R₁₂ of the formula (Va) and a group chosen from R₈ to R₁₄ of the formula (Vb) denote the group of following formula (VI): 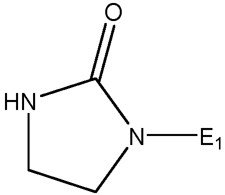 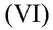 in which E₁ as defined in Claim 8; and
• the four groups of the formula (Va) chosen from R₈ to R₁₂ other than that denoting the group of formula (VI) and the six groups of the formula (Vb) chosen from R₈ to R₁₄ other than that denoting the group of formula (VI), which are identical or different, represent, independently of one another, a hydrogen atom or a preferably saturated, linear or branched aliphatic hydrocarbon chain optionally substituted or interrupted by one or more heteroatoms.

11. Modified polymer according to any one of Claims 8 to 10, in which the group E₁ of the compound of formula (V) is a preferably saturated, linear or branched C₁-C₂₄, preferably C₁-C₁₀, more preferentially C₁-C₆, hydrocarbon chain optionally interrupted by one or more nitrogen, sulfur or oxygen atoms.

12. Modified polymer according to either one of Claims 10 and 11, in which the compound of formula (V) is the compound of formula (Vb).

13. Modified polymer according to any one of Claims 8 to 12, in which the compound of formula (V) is the compound of following formula (VII):

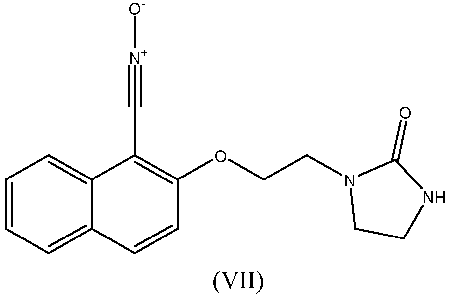

14. Composition comprising at least one modified dienic elastomer as defined in any one of Claims 1 to 13 and at least one additive.
